(19) Europäisches Patentamt
European Patent Office
Office européen des brevets

(11) Veröffentlichungsnummer: **0 185 346**
**A2**

# (12) EUROPÄISCHE PATENTANMELDUNG

(21) Anmeldenummer: 85116026.7

(22) Anmeldetag: 16.12.85

(51) Int. Cl.⁴: **C 07 D 487/04**
A 61 K 31/495, A 61 K 31/50
A 61 K 31/505, A 61 K 31/53
//(C07D487/04, 239:00, 235:00),
(C07D487/04, 253:00, 235:00),
(C07D487/04, 237:00, 235:00),
(C07D487/04, 251:00, 235:00),
(C07D487/04, 241:00, 235:00)

(30) Priorität: 21.12.84 DE 3446812

(43) Veröffentlichungstag der Anmeldung:
25.06.86 Patentblatt 86/26

(84) Benannte Vertragsstaaten:
AT BE CH DE FR GB IT LI LU NL SE

(71) Anmelder: **Dr. Karl Thomae GmbH**
**Postfach 1755**
**D-7950 Biberach (Riss)(DE)**

(72) Erfinder: **Heider, Joachim, Dr. Dipl.-Chem.**
**Am Hang 3**
**D-7951 Warthausen(DE)**

(72) Erfinder: **Austel, Volkhard, Dr. Dipl.-Chem.**
**Kapellenweg 7**
**D-7950 Biberach 1(DE)**

(72) Erfinder: **Hauel, Norbert, Dr. Dipl.-Chem.**
**Händelstrasse 12**
**D-7950 Biberach 1(DE)**

(72) Erfinder: **Noll, Klaus, Dr. Dipl.-Chem.**
**Im Schönblick 3**
**D-7951 Warthausen(DE)**

(72) Erfinder: **Bomhard, Andreas, Dr. Dipl.-Chem.**
**Dinglingerstrasse 9**
**D-7950 Biberach 1(DE)**

(72) Erfinder: **van Meel, Jacques, Dr.**
**Amriswilstrasse 7**
**D-7950 Biberach 1(DE)**

(72) Erfinder: **Diederen, Willi, Dr.**
**Haldenstrasse 1a**
**D-7950 Biberach 1(DE)**

## (54) Neue Imidazoderivate, ihre Herstellung und diese Verbindungen enthaltende Arzneimittel.

(57) Die Erfindung betrifft neue Imidazoderivate der allgemeinen Formel

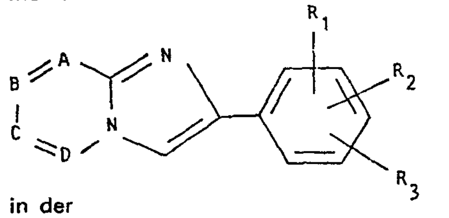

,(I)

in der

einer oder zwei der Reste A, B, C oder D ein Stickstoffatom, ein weiterer der Reste A, B, C oder D eine Hydroxymethingruppe und

die übrigen der Reste A, B, C oder D Methingruppen, wobei eine dieser Methingruppen, sofern sie neben einem Stickstoffatom steht, durch eine Hydroxymethingruppe oder durch eine durch eine Alkylmercaptogruppe substituierte Methingruppe ersetzt sein kann,

$R_1$ und $R_2$ zusammen mit zwei dazwischen liegenden Kohlenstoffatomen des Phenylringes einen gegebenenfalls durch eine Alkoxygruppe substituierten Phenlyring und

$R_3$ ein Wasserstoffatom oder eine Alkoxygruppe oder einer der Reste $R_1$, $R_2$ oder $R_3$ eine Hydroxy-, Phenyl-, alkoxy-, Alkylmercapto-, Alkylsulfinyl-, Amino-, Alkylsulfonyloxy-, Sulfamyl-, Alkylaminosulfonyl-, Dialkylaminosulfonyl-, Alkylsulfonamido-, N-Alkyl-alkylsulfonamido-, Cyano-, Aminocarbonyl-, Alkylaminocarbonyl- oder Dialkylaminocarbonylgruppe oder, wenn $R_2$ und $R_3$ nicht gleichzeitig Wasserstoffatome darstellen oder

wenn A, B, C und D zusammen mit dem Imidazolring keine Imidazo[1,2-b]pyridazin-6(5H)-one, Imidazo[1,2-c]pyrimidin-5(6H)-one und 5-Alklymecapto-imidazo[1,2-c]pyrimidin-7(8H)-one darstellen,

auch eine Alkoxy- oder Alkylsulfonylgruppe, ./...

ein zweiter der Reste $R_1$, $R_2$ oder $R_3$ ein Wasserstoffatom, eine Hydroxy- oder Alkoxygruppe und der letzte der Reste $R_1$, $R_2$ oder $R_3$ ein Wasserstoffatom oder eine Alkoxygruppe bedeuten, deren Tautomere und deren Säureadditionssalze, insbesondere deren physiologisch veträgliche Säureadditionssalze mit anorganischen oder organischen Säuren.

Die neuen Verbindungen weisen wertvolle pharmakologische Eigenschaften auf, insbesondere antithrombotische und cardiovasculäre Eigenschaften wie eine cardiotonische und/ oder eine Wirkung auf den Blutdruck, und lassen sich nach an und für sind bekannten Verfahren herstellen.

DR. KARL THOMAE GMBH
D-7950 Biberach 1

0185346
Dr.Fl./Kp.

# Neue Imidazoderivate, ihre Herstellung und diese Verbindungen enthaltende Arzneimittel

---

Gegenstand der vorliegenden Erfindung sind neue Imidazoderivate der allgemeinen Formel

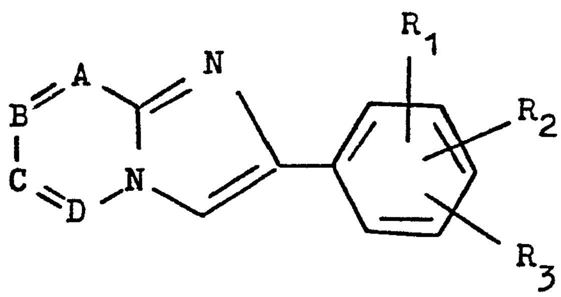

,(I)

deren Tautomere und deren Säureadditionssalze, insbesondere deren physiologisch verträgliche Säureadditionssalze mit anorganischen oder organischen Säuren, welche wertvolle pharmakologische Eigenschaften aufweisen, insbesondere antithrombotische und cardiovasculäre Eigenschaften wie eine cardiotonische und/oder eine Wirkung auf den Blutdruck.

In der obigen allgemeinen Formel I bedeutet

einer oder zwei der Reste A, B, C oder D ein Stickstoffatom,

ein weiterer der Reste A, B, C oder D eine Hydroxymethingruppe und

die übrigen der Reste A, B, C oder D Methingruppen, wobei eine dieser Methingruppen, sofern sie neben einem Stickstoffatom steht, durch eine Hydroxymethingruppe oder durch eine durch eine Alkylmercaptogruppe substituierte Methingruppe ersetzt sein kann,

$R_1$ und $R_2$ zusammen mit zwei dazwischen liegenden Kohlenstoffatomen des Phenylringes einen gegebenenfalls durch eine Alkoxygruppe substituierten Phenylring und

$R_3$ ein Wasserstoffatom oder eine Alkoxygruppe oder

einer der Reste $R_1$, $R_2$ oder $R_3$ eine Hydroxy-, Phenylalkoxy-, Alkylmercapto-, Alkylsulfinyl-, Amino-, Alkylsulfonyloxy-, Sulfamyl-, Alkylaminosulfonyl-, Dialkylaminosulfonyl-, Alkylsulfonamido-, N-Alkyl-alkylsulfonamido-, Cyano-, Aminocarbonyl-, Alkylaminocarbonyl- oder Dialkylaminocarbonylgruppe oder,

wenn $R_2$ und $R_3$ nicht gleichzeitig Wasserstoffatome darstellen oder

wenn A, B, C und D zusammen mit dem Imidazolring keine Imidazo[1,2-b]pyridazin-6(5H)-one, Imidazo[1,2-c]pyrimidin-5(6H)-one und 5-Alkylmercapto-imidazo[1,2-c]pyrimidin-7(8H)-one darstellen, auch eine Alkoxy- oder Alkylsulfonylgruppe,

ein zweiter der Reste $R_1$, $R_2$ oder $R_3$ ein Wasserstoffatom, eine Hydroxy- oder Alkoxygruppe und

der letzte der Reste $R_1$, $R_2$ oder $R_3$ ein Wasserstoffatom oder eine Alkoxygruppe, wobei der Alkylteil bei allen vorstehend erwähnten Resten 1 oder 2 Kohlenstoffatome enthalten kann.

Gegenstand der vorliegenden Erfindung sind somit insbesondere die neuen Imidazo[1.2-a]pyrimidin-7-one und -5,7-dione, Imidazo[1.2-c]pyrimidin-5-one und -7-one, Imidazo[2.1-f]-[1.2.4]triazin-2-one, -4-one und -2,4-dione, Imidazo[1.2-b]-pyridazin-6-one, Imidazo[1.2-a][1.3.5]triazin-2-one, -4-one und -2,4-dione, Imidazo[1.2-a]pyrazin-6-one und -8-one, Imidazo[1.2-d][1.2.4]triazin-5-one, -8-one und -5,8-dione, Imidazo[1.2-b][1.2.4]triazin-2-one und -3-one sowie Imidazo[2.1-c][1.2.4]triazin-3-one, deren Tautomere und deren Säureadditionssalze, insbesondere deren physiologisch verträgliche Säureadditionssalze.

Für die bei der Definition der Reste $R_1$ bis $R_3$ eingangs erwähnten Bedeutungen kommt somit

für $R_1$ und $R_2$ zusammen mit dem Phenylring insbesondere die Bedeutung der Naphth-1-yl-, Naphth-2-yl-, 2-Methoxy-naphth-1-yl-, 3-Methoxy-naphth-1-yl-, 4-Methoxy-naphth-1-yl-, 1-Methoxy-naphth-2-yl-, 3-Methoxy-naphth-2-yl-, 4-Methoxy-naphth-2-yl-, 5-Methoxy-naphth-2-yl-, 6-Methoxy-naphth-2-yl-, 7-Methoxy-naphth-2-yl- oder 8-Methoxy-naphth-2-yl-gruppe und

für $R_3$ die des Wasserstoffatoms, der Methoxy- oder Ethoxygruppe oder

für $R_1$ die der Hydroxy-, Methoxy-, Ethoxy-, Benzyloxy-, 1-Phenylethoxy-, 2-Phenylethoxy-, Methylmercapto-, Ethylmercapto-, Methylsulfinyl-, Ethylsulfinyl-, Methylsulfonyl-, Ethylsulfonyl-, Amino-, Methylsulfonyloxy-, Ethylsulfonyloxy-, Sulfamyl-, Methylaminosulfonyl-, Ethylaminosulfonyl-, Dimethylaminosulfonyl-, Diethylaminosulfonyl-, N-Methylethylaminosulfonyl-, Methylsulfonamido-, Ethylsulfonamido-, N-Methyl-methylsulfonamido-, N-Ethyl-methylsulfonamido-, N-Methyl-ethylsulfonamido-, N-Ethyl-ethylsulfonamido-, Cyano-, Aminocarbonyl-, Methylaminocarbonyl-, Ethylaminocarbonyl-, Dimethylaminocarbonyl-, Diethylaminocarbonyl- oder N-Methyl-ethylaminocarbonylgruppe,

für $R_2$ die des Wasserstoffatoms, der Methoxy- oder Ethoxygruppe und

für $R_3$ die des Wasserstoffatoms, der Methoxy- oder Ethoxygruppe in Betracht.

Bevorzugte Verbindungen der obigen allgemeinen Formel I sind diejenigen, in denen

ein oder zwei der Reste A, B, C oder D ein Stickstoffatom,

ein weiterer der Reste A, B, C oder D eine Hydroxymethingruppe und

die übrigen der Reste A, B, C oder D Methingruppen, wobei eine dieser Methingruppen, sofern sie neben einem Stickstoffatom steht, durch eine Hydroxymethingruppe ersetzt sein kann,

$R_1$ und $R_2$ zusammen mit zwei dazwischenliegenden Kohlenstoffatomen des Phenylringes einen Phenyl- oder Methoxyphenylring und $R_3$ ein Wasserstoffatom oder eine Methoxygruppe oder

einer der Reste $R_1$, $R_2$ oder $R_3$ eine Hydroxy-, Benzyloxy-, Methylmercapto-, Methylsulfinyl-, Methylsulfonyl-, Amino-, Methylsulfonyloxy-, Methylsulfonamido-, N-Methyl-methylsulfonamido-, Sulfamyl-, Methylaminosulfonyl-, Dimethylaminosulfonyl-, Cyano-, Aminocarbonyl-, Methylaminocarbonyl- oder Dimethylaminocarbonylgruppe oder,

wenn $R_2$ und $R_3$ nicht gleichzeitig Wasserstoffatome bedeuten oder

wenn A, B, C und D zusammen mit dem Imidazolring keine Imidazo[1,2-b]pyridazine-6(5H)-one und Imidazo[1,2-c]pyrimidin-5(6H)-one darstellen,

auch eine Methoxygruppe,

ein zweiter der Reste $R_1$, $R_2$ oder $R_3$ ein Wasserstoffatom oder eine Methoxygruppe und

der letzte der Reste $R_1$, $R_2$ oder $R_3$ ein Wasserstoffatom oder eine Methoxygruppe bedeuten, insbesondere jedoch diejenigen Verbindungen, in denen $R_1$ in 4-Position und $R_2$ in 2-Position steht, deren Tautomere und deren Säureadditionssalze, insbesondere deren physiologisch verträgliche Säureadditionssalze.

Besonders bevorzugte Verbindungen der obigen allgemeinen Formel I sind jedoch diejenigen, in denen

A, B, C oder D wie vorstehend erwähnt definiert sind,

$R_1$ in 4-Stellung eine Cyano-, Dimethylaminocarbonyl-, Methylsulfonyloxy-, Methylsulfonamido-, N-Methyl-methylsulfonamido-, Sulfamyl-, Methylaminosulfonyl- oder Dimethylaminosulfonylgruppe, oder,

wenn $R_2$ und $R_3$ nicht gleichzeitig Wasserstoffatome bedeuten oder

wenn A, B, C und D zusammen mit dem Imidazolring keine Imidazo[1,2-b]pyridazine-6(5H)-one und Imidazo[1,2-c]pyrimidin-5(6H)-one darstellen,

auch eine Methoxygruppe,

$R_2$ in 2-Stellung eine Methoxygruppe und

$R_3$ ein Wasserstoffatom darstellen, deren Tautomere und deren Säureadditionssalze, insbesondere deren physiologisch verträgliche Säureadditionssalze.

Erfindungsgemäß erhält man die neuen Verbindungen nach folgenden Verfahren:

a) Umsetzung einer Verbindung der allgemeinen Formel

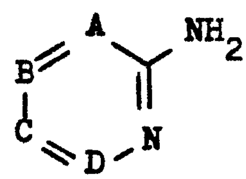

,(II)

in der
A, B, C und D wie eingangs definiert sind, mit einer Verbindung der allgemeinen Formel

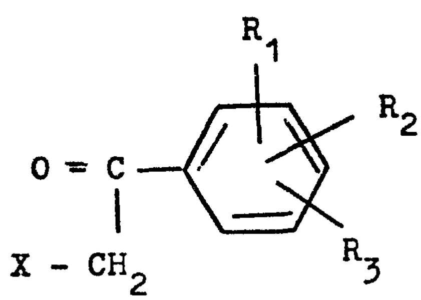

,(III)

in der
$R_1$, $R_2$ und $R_3$ wie eingangs definiert sind und
X eine nukleophile Austrittsgruppe wie ein Halogenatom, z.B. ein Chlor- oder Bromatom, darstellt.

Die Umsetzung wird zweckmäßigerweise in einem Lösungsmittel oder Lösungsmittelgemisch wie Ethanol, Isopropanol, Benzol, Glycol, Glycolmonomethylether, Dimethylformamid oder Dioxan beispielsweise bei Temperaturen zwischen 0 und 150°C, vorzugsweise bei Temperaturen zwischen 20 und 100°C, durchgeführt. Die Umsetzung kann jedoch auch ohne Lösungsmittel durchgeführt werden.

b) Zur Herstellung von Verbindungen der allgemeinen Formel I, in der einer der Reste $R_1$, $R_2$ oder $R_3$ eine Alkylsulfonyloxy-, Alkylsulfonamido- oder N-Alkyl-alkylsulfonamidogruppe darstellt:

Umsetzung einer Verbindung der allgemeinen Formel

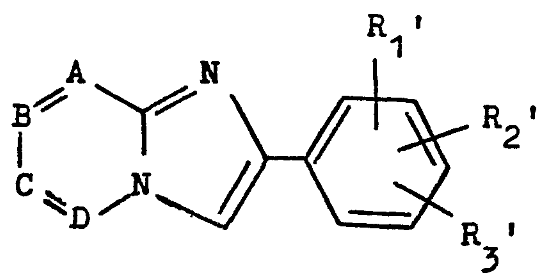

,(IV)

in der
A, B, C und D wie eingangs definiert sind und
die Reste $R_1'$, $R_2'$ und $R_3'$ mit Ausnahme der Alkylsulfonyloxy-, Alkylsulfonamido- und N-Alkyl-alkylsulfonamidogruppe die für $R_1$, $R_2$ oder $R_3$ eingangs erwähnten Bedeutungen besitzen, wobei jedoch einer der Reste $R_1'$, $R_2'$ oder $R_3'$ eine Hydroxy-, Amino-, Methylamino- oder Ethylaminogruppe darstellen muß, mit einer Verbindung der allgemeinen Formel

$$R_4 - SO_2 - Y \qquad ,(V)$$

in der
$R_4$ eine Methyl- oder Ethylgruppe und
Y eine nukleophile Austrittsgruppe wie ein Halogenatom oder eine Alkoxygruppe, z.B. ein Chlor- oder Bromatom, eine Methoxy-, Ethoxy- oder Benzyloxygruppe, bedeuten.

Die Umsetzung wird zweckmäßigerweise in einem Lösungsmittel oder Lösungsmittelgemisch wie Wasser, Methanol, Ethanol, Isopropanol, Methylenchlorid, Ether, Tetrahydrofuran,

Dioxan, Dimethylformamid oder Benzol gegebenenfalls in Gegenwart eines säurebindenden Mittels wie Natriumcarbonat, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

c) Zur Herstellung von Verbindungen der allgemeinen Formel I, in der mindestens einer der Reste $R_1$, $R_2$ oder $R_3$ eine Alkoxy-, Phenylalkoxy- oder Alkylmercaptogruppe oder einer der Reste $R_1$, $R_2$ oder $R_3$ eine N-Alkyl-alkylsulfonamidogruppe darstellen:

Alkylierung einer Verbindung der allgemeinen Formel

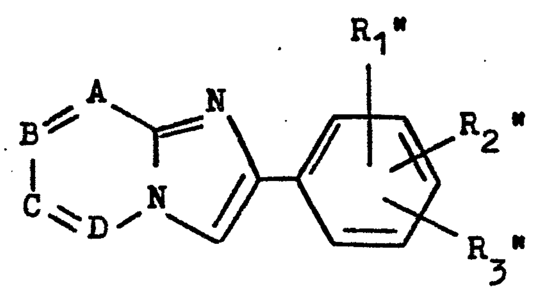

,(VI)

in der

A, B, C und D wie eingangs definiert sind und

$R_1''$, $R_2''$ und $R_3''$ die für $R_1$, $R_2$ oder $R_3$ eingangs erwähnten Bedeutungen besitzen, wobei jedoch mindestens einer der Reste $R_1''$, $R_2''$ oder $R_3''$ eine Hydroxy-, Mercapto- oder Alkylsulfonamidogruppe darstellen muß, mit einer Verbindung der allgemeinen Formel

$$R_4 - Z \qquad ,(VII)$$

in der

$R_4$ eine Alkyl- oder Phenylalkylgruppe mit jeweils 1 oder 2 Kohlenstoffatomen im Alkylteil und

Z eine nukleophile Austrittsgruppe wie ein Halogenatom oder eine Sulfonyloxygruppe, z.B. ein Chlor-, Brom- oder Jodatom, eine Methylsulfonyloxy-, Methoxysulfonyloxy- oder p-Tolylsulfonyloxygruppe, darstellen.

Die Umsetzung wird mit einem Alkylierungsmittel wie Methyljodid, Ethyljodid, Dimethylsulfat oder p-Toluolsulfonsäuremethylester zweckmäßigerweise in einem Lösungmittel wie Tetrahydrofuran, Dioxan, Dimethylformamid, Sulfolan, Dimethylsulfoxid oder Ethylenglycoldimethylether gegebenenfalls in Gegenwart eines säurebindenden Mittels wie Kaliumkarbonat, Kalium-tert.butylat, Triethylamin oder Pyridin, wobei die beiden letzteren gleichzeitig auch als Lösungsmittel verwendet werden können, bei Temperaturen zwischen 0 und 100°C, vorzugsweise bei Temperaturen zwischen 20 und 50°C, durchgeführt.

d) Zur Herstellung von Verbindungen der allgemeinen Formel I, in der einer der Reste $R_1$, $R_2$ oder $R_3$ eine Aminocarbonyl-, Alkylaminocarbonyl-, Dialkylaminocarbonyl-, Sulfamyl-, Alkylaminosulfonyl- oder Dialkylaminosulfonylgruppe darstellt:

Umsetzung einer Verbindung der allgemeinen Formel

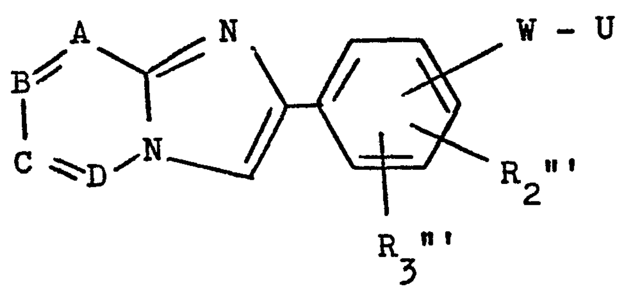

,(VIII)

in der
A, B, C und D wie eingangs definiert sind,
$R_2'''$ und $R_3'''$ die für $R_1$, $R_2$ und $R_3$ mit Ausnahme der Aminocarbonyl-, Alkylaminocarbonyl-, Dialkylaminocarbonyl-, Sulfamyl-, Alkylaminosulfonyl- oder Dialkylaminosulfonylgruppe eingangs erwähnten Bedeutungen besitzen,
W eine Carbonyl- oder Sulfonylgruppe und
U eine nukleophile Austrittsgruppe wie ein Halogenatom oder eine Alkoxygruppe, z.B. ein Chloratom, eine Methoxy- oder Ethoxygruppe, darstellen, mit einem Amin der allgemeinen Formel

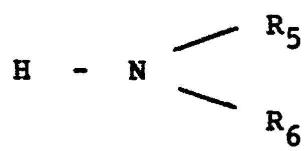

,(IX)

in der
$R_5$ und $R_6$, die gleich oder verschieden sein können, jeweils ein Wasserstoffatom, eine Methyl- oder Ethylgruppe darstellen.

Die Umsetzung wird zweckmäßigerweise in einem Lösungsmittel oder Lösungsmittelgemisch wie Wasser, Methanol, Ethanol, Isopropanol, Methylenchlorid, Ether, Tetrahydrofuran, Dioxan, Dimethylformamid oder Benzol gegebenenfalls in Gegenwart eines säurebindenden Mittels wie Natriumcarbonat, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

Bedeutet W eine Carbonylgruppe, so kann U auch eine Hydroxygruppe bedeuten. In diesem Falle wird die Umsetzung vorzugsweise in Gegenwart eines wasserentziehenden Mittels, z.B. in Gegenwart von Chlorameisensäureethylester, Thionylchlorid, Phosphortrichlorid, Phosphorpentoxid, N,N'-Dicyclohexylcar-

bodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triethylamin oder Pyridin, welche gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels durchgeführt werden, desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

e) Zur Herstellung von Verbindungen der allgemeinen Formel I, in der die Reste A, B, C und D wie eingangs definiert sind und einer der Reste $R_1$, $R_2$ oder $R_3$ eine Amino-, Hydroxy- oder Aminocarbonylgruppe darstellt oder $R_1$, $R_2$ und $R_3$ wie eingangs definiert sind und mindestens einer der Reste A, B, C oder D eine Hydroxymethingruppe darstellt:

Hydrolyse einer Verbindung der allgemeinen Formel

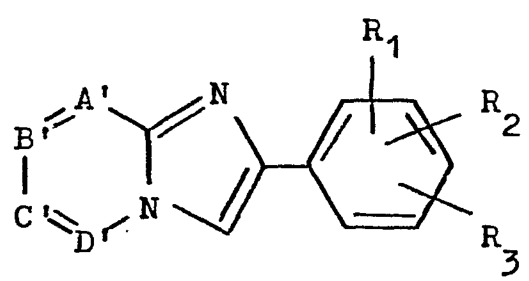

,(X)

in der

$R_1$, $R_2$ und $R_3$ wie eingangs definiert sind und A', B', C' und D' die für A, B, C und D eingangs erwähnten Bedeutungen aufweisen, wobei jedoch entweder eine der eingangs erwähnten Methingruppen durch einen hydrolytisch abspaltbaren Rest wie durch ein Halogenatom, eine Alkoxy- oder

Alkylmercaptogruppe, z.B. ein Chlor- oder Bromatom, eine Methoxy- oder Methylmercaptogruppe, substituiert ist oder einer der Reste $R_1$, $R_2$ oder $R_3$ eine Alkanoylamino-, Cyano- oder Alkylsulfonyloxygruppe darstellen muß.

Die Umsetzung wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid oder dem Alkalisalz eines entsprechenden Alkohols in der Schmelze oder in einem geeigneten Lösungmittel wie Wasser, Wasser/Methanol, Ethanol, Wasser/Ethanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

Die partielle Hydrolyse der Cyanogruppe wird jedoch vorzugsweise mit konzentrierter Schwefelsäure oder mit einer Alkalilauge in Gegenwart von Wasserstoffperoxid, z.B. mit Natronlauge/Wasserstoffperoxid, zweckmäßigerweise bei Raumtemperatur durchgeführt.

f) Zur Herstellung von Verbindungen der allgemeinen Formel I, in der
einer oder zwei der Reste A, B, C oder D ein Stickstoffatom, ein weiterer der Reste A, B, C oder D eine Hydroxymethin- oder Alkylmercaptomethingruppe und
die übrigen der Reste A, B, C oder D Methingruppen darstellen:

Reduktive Abspaltung eines oder zweier Reste von einer Verbindung der allgemeinen Formel

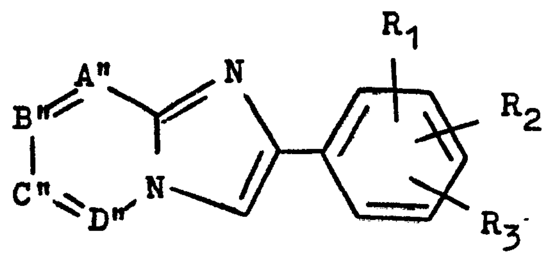

,(XI)

in der

$R_1$, $R_2$ und $R_3$ wie eingangs definiert sind und die Reste A", B", C" und D" die für A, B, C und D eingangs erwähnten Bedeutungen aufweisen, wobei jedoch mindestens eine der eingangs erwähnten Methingruppen durch einen reduktiv abspaltbaren Rest wie durch ein Halogenatom oder eine Alkylmercaptogruppe substituiert sein muß.

Die reduktive Abspaltung erfolgt vorzugsweise mittels Hydrogenolyse. Diese wird zweckmäßigerweise in einem Lösungsmittel wie Methanol, Ethanol, Isopropanol, Eisessig, Essigester, Dimethylformamid oder Wasser gegebenenfalls in Gegenwart einer Mineralsäure wie Salzsäure oder Bromwasserstoffsäure bei Temperaturen zwischen -10°C und 100°C, vorzugsweise bei 0°C bis 60°C in Gegenwart eines Katalysators wie Raney-Nickel, Platin, Platinoxid- oder Palladium auf Kohle durchgeführt. - Eine gegebenenfalls vorhandene Benzyloxygruppe wird hierbei während der Umsetzung in eine Hydroxygruppe übergeführt.

Erhält man erfindungsgemäß eine Verbindung der allgemeinen Formel I, in der einer der Rest $R_1$, $R_2$ oder $R_3$ eine Aminogruppe darstellt, so kann diese über das entsprechende Diazoniumsalz in eine Verbindung der allgemeinen Formel I, in der einer der Reste $R_1$, $R_2$ oder $R_3$ eine Cyanogruppe darstellt, übergeführt werden.

Die nachträgliche Umsetzung eines Diazoniumsalzes, z.B. des Hydrosulfats in Schwefelsäure oder des Hydrochlorids, in Gegenwart des entsprechenden Kupfer-(I)-Salzes wie Kupfer-(I)-cyanid/Salzsäure, wird bei leicht erhöhten Temperaturen, z.B. bei Temperaturen zwischen 15°C und 100°C, durchgeführt. Das erforderliche Diazoniumsalz wird zweckmäßigerweise in einem geeigneten Lösungsmittel, z.B. in Wasser/Salzsäure, Methanol/Salzsäure, Äthanol/Salzsäure oder Dioxan/Salzsäure, durch Diazotierung einer entsprechenden Aminoverbindung mit einem Nitrit, z.B. Natriumnitrit oder einem Ester der salpetrigen Säure, bei niedrigen Temperaturen, z.B. bei Temperaturen zwischen -10°C und 5°C, hergestellt.

Ferner können die erfindungsgemäß erhaltenen Verbindungen der allgemeinen Formel I gewünschtenfalls in ihre physiologisch verträglichen Säureadditionssalze mit anorganischen oder organischen Säuren übergeführt werden. Als Säuren kommen hierfür beispielsweise Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure in Betracht.

Die als Ausgangsstoffe verwendeten Verbindungen der allgemeinen Formeln II bis XI sind teilweise literaturbekannt bzw. erhält man nach literaturbekannten Verfahren.

So erhält man beispielsweise die als Ausgangsstoffe verwendeten Verbindungen der allgemeinen Formeln IV, VI, VIII, X und XI durch Umsetzung einer entsprechenden Aminoverbindung mit einem entsprechenden α-Bromacetophenon und gegebenenfalls anschließende Chlorsulfonierung.

Wie bereits eingangs erwähnt weisen die neuen Verbindungen der allgemeinen Formel I, deren 1H Tautomere und deren physiologisch verträgliche Säureadditionssalze überlegene pharmakologische Eigenschaften auf, insbesondere antithrombotische und cardiovasculäre Eigenschaften wie eine cardiotonische und/oder eine Wirkung auf den Blutdruck.

Beispielsweise wurden die Verbindungen

A = 2-(4-Dimethylaminosulfonyl-phenyl)-8H-imidazo[1,2-a]-pyrimidin-7-on,

B = 2-(4-Methoxy-phenyl)-7H-imidazo[1,2-a]pyrazin-8-on,

C = 7-(4-Methoxy-phenyl)-1H,3H-imidazo[1,2-a][1.3.5]triazin-2,4-dion,

D = 2-(2-Methoxy-4-methylsulfonyloxy-phenyl)-8H-imidazo-[1,2-a]pyrimidin-7-on,

E = 2-(2-Methoxy-4-cyano-phenyl)-8H-imidazo[1,2-a]pyrimidin-7-on,

F = 2-(2-Methoxy-4-dimethylaminosulfonyl-phenyl)-8H-imidazo-[1,2-a]pyrimidin-7-on,

G = 2-(2-Methoxy-4-methylaminosulfonyl-phenyl)-8H-imidazo-[1,2-a]pyrimidin-7-on,

H = 2-(2-Methoxy-4-dimethylaminocarbonyl-phenyl)-8H-imidazo-[1,2-a]pyrimidin-7-on und

I = 2-(2-Methoxy-4-methylsulfonyloxy-phenyl)-7H-imidazo-[1,2-a]pyrazin-8-on

auf ihre biologischen Eigenschaften wie folgt untersucht:

Bestimmung der positiv inotropen Wirkung an despinalisierten Meerschweinchen

Die Untersuchungen wurden an despinalisierten Meerschweinchen durchgeführt. Die Tiere wurden mit 50 % $O_2$ + 50 % $N_2$ beatmet. Der arterielle Blutdruck wurde in der rechten Arteria Carotis mit einem Bell and Howell Druckwandler (4-327-I) gemessen. Für die Erfassung der positiv inotropen Wirkung wurde mit einem Kathetertipmanometer (Millar PR-249) der Druck in dem linken Ventrikel (LV) gemessen. Mittels eines Differenzierers wurde LV-dp/dtmax gewonnen. Die zu untersuchenden Substanzen wurden in eine Vena jugularis injiziert. Als Lösungsmittel diente 0,9 % NaCl+Polydiol 200 (1:1).

Jede Substanz wurde an 3 Meerschweinchen überprüft. Die Dosen waren 0,1 - 3 mg/kg i.v.. Die nachfolgende Tabelle enthält die Mittelwerte bei 1 mg/kg i.v..

| Substanz | Dosis mg/kg i.v. | Zunahme in LV-dp/dtmax |
|---|---|---|
| A | 1 | 32 % |
| B | 1 | 45 % |
| C | 1 | 86 % |
| D | 1 | 63 % |
| E | 1 | 44 % |
| F | 1 | 47 % |
| G | 1 | 75 % |
| H | 1 | 33 % |
| I | 1 | 98 % |

Die neuen Verbindungen sind gut verträglich, so konnte bei der Untersuchung der Substanzen A bis I bis zu einer Dosis von 3 mg/kg i.v. keinerlei herztoxische Wirkungen bzw. Kreislaufschäden beobachtet werden.

Aufgrund ihrer pharmakologischen Eigenschaften eignen sich die erfindungsgemäß hergestellten Verbindungen der allgemeinen Formel I sowie deren physiologisch verträgliche Säureadditionssalze zur Behandlung von Herzinsuffizienzen unterschiedlicher Genese, da sie die Kontraktionskraft des Herzens steigern und zum Teil durch Blutdrucksenkung die Entleerung des Herzens erleichtern.

Hierzu lassen sich die neuen Verbindungen sowie deren physiologisch verträgliche Säureadditionssalze, gegebenenfalls in Kombination mit anderen Wirksubstanzen, in die üblichen pharmazeutischen Anwendungsformen wie Tabletten, Dragées, Pulver, Suppositorien, Suspensionen, Ampullen oder Tropfen einarbeiten. Die Einzeldosis beträgt hierbei 1-4 x täglich 0,1 - 15 mg/kg Körpergewicht, vorzugsweise jedoch 3 - 10 mg/kg Körpergewicht.

Die nachfolgenden Beispiele sollen die Erfindung näher erläutern:

Beispiel 1

2-(4-Methylsulfonyloxy-phenyl)-8H-imidazo[1,2-a]pyrimidin-7-on

2,2 g (0,02 Mol) 2-Amino-4-hydroxy-pyrimidin und 5,9 g (0,02 Mol) α-Brom-4-methylsulfonyloxy-acetophenon werden in 50 ml Dimethylformamid bei Raumtemperatur gerührt. Nach 6 Stunden wird die klare Lösung im Vakuum zur Trockne eingedampft und das erhaltene Rohprodukt über eine Säule (Kieselgel, Korngröße: 0,2-0,5 mm, Elutionsmittel= Methylenchlorid:Äthanol-Gemische) gereinigt. Das gewünschte Endprodukt wird aus Äthanol/Dimethylformamid umkristallisiert.

Ausbeute: 37,7 % der Theorie,

Schmelzpunkt: 246-248°C

| | | | | | | | | |
|---|---|---|---|---|---|---|---|---|
| Ber.: | C | 51,14 | H | 3,63 | N | 13,76 | S | 10,50 |
| Gef. | | 51,06 | | 3,58 | | 13,76 | | 10,70 |

Beispiel 2

6-(4-Methylsulfonyloxy-phenyl)-3H-imidazo[2,1-f][1.2.4]-triazin-4-on

425 mg (1,86 mMol) 6-(4-Hydroxyphenyl)-3H-imidazo[2,1-f]-[1,2,4]triazin-4-on werden in 20 ml 1n Natronlauge gelöst. Bei Raumtemperatur werden 456 mg Methansulfonsäurechlorid zugegeben und 2 Stunden bei Raumtemperatur nachgerührt. Die hellgelbe klare Lösung wird in der Kälte mit 2n Salzsäure neutralisiert. Der ausgefallene weißgraue Niederschlag wird abgesaugt, mit Wasser gewaschen, getrocknet und in Methylenchlorid/Äthanol = 1:1 gelöst; es werden 10 g Kieselgel zugesetzt und zur Trockne eingedampft, der Eindampfrückstand

wird auf eine Kieselgelsäule gegeben und zuerst mit Methylenchlorid, später mit Methylenchlorid/Äthanol = 50:1 und 25:1 eluiert. Die gewünschten Eluate werden eingeengt, der kristalline Rückstand mit Äther verrieben, abgesaugt und im Vakuum bei 50°C getrocknet.
Ausbeute: 9,65 % der Theorie,
Schmelzpunkt: 304-306°C

| | | | | | | | | |
|---|---|---|---|---|---|---|---|---|
| Ber.: | C | 47,07 | H | 3,29 | N | 18,30 | S | 10,45 |
| Gef. | | 47,44 | | 3,58 | | 18,04 | | 10,06 |

Beispiel 3

2-(3-Dimethylaminosulfonyl-4-methoxy-phenyl)-6H-imidazo[1,2-c]pyrimidin-5-on

a) 2-(3-Chlorsulfonyl-4-methoxy-phenyl)-6H-imidazo[1,2-c]-pyrimidin-5-on

1,5 g (6,2 mMol) 2-(4-Methoxy-phenyl)-6H-imidazo[1,2-c]pyrimidin-5-on werden bei 0-5°C unter Rühren in 15 ml Chlorsulfonsäure eingetragen. Nach 3-stündigem Stehen bei Raumtemperatur wird die Reaktionsmischung in Eiswasser gegossen und das ausgefallene Produkt abgesaugt und getrocknet.

b) 2-(3-Dimethylaminosulfonyl-4-methoxy-phenyl)-6H-imidazo-[1,2-c]pyrimidin-5-on

1,5 g (4,4 mMol) des unter a) erhaltenen Produktes werden in 30 ml wässriger Dimethylaminlösung (40%ig) aufgenommen und auf 50°C erwärmt. Nach 30 Minuten wird die klare Lösung mit Wasser verdünnt, mit Essigester extrahiert und die vereinigten Extrakte nach Trocknen über Natriumsulfat im Vakuum eingedampft. Das erhaltene Rohprodukt wird aus Äthanol kristallisiert.

Ausbeute: 73,3 % der Theorie,
Schmelzpunkt: 288-290°C

| | | | | | | | | |
|---|---|---|---|---|---|---|---|---|
| Ber.: | C | 51,71 | H | 4,63 | N | 16,08 | S | 9,20 |
| Gef. | | 51,69 | | 4,57 | | 16,24 | | 9,25 |

## Beispiel 4

2-(4-Hydroxy-2-methoxy-phenyl)-5H-imidazo[1,2-b]-pyridazin-6-on

0,4 g (1,13 mMol) 6-Chlor-2-(2-methoxy-4-methylsulfonyloxy-phenyl)-imidazo[1,2-b]pyridazin wird 6,5 Stunden lang in 30 ml 4n Kalilauge zum Rückfluß erhitzt. Nach Filtrieren der heißen Lösung erhält man das Produkt durch Ansäuern mit konzentrierter Salzsäure.

Ausbeute: 100% der Theorie,
Schmelzpunkt: > 250°C
NMR-Spektrum (Dimethylsulfoxid/Deuteromethanol):
$OCH_3$: 3,95 ppm (s) 3H

| | | | | |
|---|---|---|---|---|
| H am Phenylring: | 6,65 | ppm | (dd) | 1H |
| | 6,7 | ppm | (d) | 1H |
| | 7,82 | ppm | (d) | 1H |
| H in 3-Stellung: | 8,45 | ppm | (s) | 1H |
| 7-Stellung: | 8,3 | ppm | (d) | 1H |
| 8-Stellung: | 7,4 | ppm | (d) | 1H |

| | |
|---|---|
| UV-Spektrum (Äthanol): | 256 nm (0,19) |
| | 352 nm (0,19) |
| (Äthanol + KOH): | 275 nm (0,26) |
| | 370 nm (0,19) |

Beispiel 5

6-(4-Hydroxy-phenyl)-3H-imidazo[2,1-f][1,2,4]triazin-4-on

1,37 g (0,005 mMol) 6-(4-Hydroxy-phenyl)-2-methylmercapto-3H-imidazo[2,1-f][1,2,4]triazin-4-on wird in 60 ml Äthanol gelöst, mit 5 g Raney-Nickel versetzt und 7 Stunden in einer Parr-Apparatur bei 80°C bei einem Wasserstoffdruck von 5 bar geschüttelt. Anschließend wird vom Katalysator abfiltriert, mit 20 g Kieselgel versetzt, eingedampft und der erhaltene Rückstand über eine Kieselgelsäule gereinigt. Zuerst wird mit Methylenchlorid, später mit Methylenchlorid/Äthanol = 50:1 und 25:1 eluiert. Die die gewünschte Substanz enthaltende Eluate werden eingeengt, der kristalline Rückstand wird mit Äther verrieben, abgesaugt und im Vakuum bei 50°C getrocknet.

Ausbeute: 18,4 % der Theorie,

Schmelzpunkt: 314-316°C

| | | | | | | |
|---|---|---|---|---|---|---|
| Ber.: | C | 57,89 | H | 3,53 | N | 24,55 |
| Gef.: | | 57,70 | | 3,74 | | 24,50 |

Beispiel 6

2-(4-Dimethylaminosulfonylphenyl)-8H-imidazo[1,2-a]pyrimidin-7-on

Hergestellt aus 2-Amino-4-hydroxy-pyrimidin und α-Brom-4-dimethylaminosulfonyl-acetophenon analog Beispiel 1.

Ausbeute: 27,3 % der Theorie,

Schmelzpunkt: 300-302°C

| | | | | | | | | |
|---|---|---|---|---|---|---|---|---|
| Ber.: | C | 52,82 | H | 4,43 | N | 17,60 | S | 10,07 |
| Gef.: | | 52,76 | | 4,65 | | 17,60 | | 10,27 |

Beispiel 7

2-(4-Dimethylaminosulfonyl-phenyl)-6H-imidazo[1,2-c]pyrimidin-5-on

Hergestellt aus 2-Hydroxy-4-amino-pyrimidin und α-Brom-4-dimethylaminosulfonyl-acetophenon analog Beispiel 1.
Ausbeute: 28,7 % der Theorie,
Schmelzpunkt: 246-248°C

| | | | | | | | | |
|---|---|---|---|---|---|---|---|---|
| Ber.: | C | 52,82 | H | 4,43 | N | 17,60 | S | 10,07 |
| Gef.: | | 52,41 | | 4,51 | | 17,55 | | 10,19 |

Beispiel 8

2-(4-Methoxy-phenyl)-8H-imidazo[1,2-a]pyrimidin-7-on

Hergestellt aus 2-Amino-4-hydroxy-pyrimidin und α-Brom-4-methoxy-acetophenon analog Beispiel 1.
Ausbeute: 29,2 % der Theorie,
Schmelzpunkt: 290-295°C

| | | | | | | |
|---|---|---|---|---|---|---|
| Ber.: | C | 64,72 | H | 4,60 | N | 17,42 |
| Gef.: | | 64,60 | | 4,60 | | 17,16 |

Beispiel 9

2-(4-Methylsulfonamido-phenyl)-6H-imidazo[1,2-c]pyrimidin-5-on

Hergestellt aus 2-Hydroxy-4-amino-pyrimidin und α-Brom-4-methylsulfonamido-acetophenon analog Beispiel 1.
Ausbeute: 34,6 % der Theorie,
Schmelzpunkt: 293-295°C

| | | | | | | | | |
|---|---|---|---|---|---|---|---|---|
| Ber.: | C | 49,44 | H | 4,37 | N | 17,38 | S | 10,54 |
| Gef.: | | 49,41 | | 4,10 | | 17,00 | | 10,71 |

Beispiel 10

2-(4-Methylsulfonyloxy-phenyl)-6H-imidazo[1,2-c]pyrimidin-5-on

Hergestellt aus 2-Hydroxy-4-amino-pyrimidin und α-Brom-4-methylsulfonyloxy-acetophenon analog Beispiel 1.
Ausbeute: 29,6 % der Theorie,
Schmelzpunkt: 302-305°C

| | | | | | | | | |
|---|---|---|---|---|---|---|---|---|
| Ber.: | C | 51,14 | H | 3,63 | N | 13,76 | S | 10,50 |
| Gef.: | | 50,88 | | 3,70 | | 13,68 | | 10,53 |

Beispiel 11

2-(4-Methoxy-phenyl)-6H-imidazo[1,2-c]pyrimidin-5-on

Hergestellt aus 2-Hydroxy-4-amino-pyrimidin und α-Brom-6-methoxy-acetophenon analog Beispiel 1.
Ausbeute: 36,9 % der Theorie,
Schmelzpunkt: 255-258°C

| | | | | | | |
|---|---|---|---|---|---|---|
| Ber.: | C | 64,72 | H | 4,60 | N | 17,42 |
| Gef.: | | 64,81 | | 4,60 | | 17,67 |

Beispiel 12

7-(4-Methylsulfonamido-phenyl)-1H,3H-imidazo[1,2-a][1,3,5]-triazin-2,4-dion

Hergestellt aus 6-Amino-1H,3H-[1,3,5]triazin-2,4-dion und α-Brom-4-methylsulfonamido-acetophenon analog Beispiel 1.
Ausbeute: 28 % der Theorie,
Schmelzpunkt: > 300°C
$R_f$-Wert: 0,60 (Methylenchlorid/Methanol = 7:3; $SiO_2$)
Massenspektrum: $M^+$: 321 m/e
UV-Spektrum(Ethanol): 278 nm (0,18)

Beispiel 13

7-(4-Methoxy-phenyl)-1H,3H-imidazo[1,2-a][1,3,5]triazin-2,4-dion

Hergestellt aus 6-Amino-1H,3H-[1,3,5]triazin-2,4-dion und α-Brom-4-methoxy-acetophenon analog Beispiel 1.
Ausbeute: 31 % der Theorie,
Schmelzpunkt: > 300°C
$R_f$-Wert: 0,70 (Methylenchlorid/Methanol = 7:3; $SiO_2$)
Massenspektrum: $M^+$: 258 m/e
UV-Spektrum (Ethanol): 274 nm (0,37)

Beispiel 14

2-(4-Methylsulfonamido-phenyl)-7H-imidazo[1,2-a]pyrazin-8-on

Hergestellt aus 3-Amino-1H-pyrazin-2-on und α-Brom-4-methylsulfonamido-acetophenon analog Beispiel 1.

Ausbeute: 15 % der Theorie,

Schmelzpunkt: 323°C

| | | | | | | | | |
|---|---|---|---|---|---|---|---|---|
| Ber.: | C | 51,31 | H | 3,98 | N | 18,12 | S | 10,54 |
| Gef.: | | 50,97 | | 4,32 | | 18,12 | | 10,78 |

Beispiel 15

2-(4-Methoxy-phenyl)-7H-imidazo[1,2-a]pyrazin-8-on

Hergestellt aus 3-Amino-1H-pyrazin-2-on und α-Brom-4-methoxy-acetophenon analog Beispiel 1.

Ausbeute: 25 % der Theorie,

Schmelzpunkt: > 300°C

$R_f$-Wert: 0,65 (Acetonitril/Ameisensäure = 9:1; $SiO_2$)

| | | | | | | |
|---|---|---|---|---|---|---|
| Ber.: | C | 64,72 | H | 4,60 | N | 17,42 |
| Gef.: | | 65,00 | | 4,65 | | 16,43 |

Beispiel 16

2-(4-Methylsulfonyloxy-phenyl)-6H-imidazo[1,2-d][1,2,4]-triazin-5-on

Hergestellt aus 5-Amino-2H-[1,2,4]triazin-3-on und α-Brom-4-methylsulfonyloxy-acetophenon in Dimethylformamid analog Beispiel 1.

Ausbeute: 40,2 % der Theorie,

Schmelzpunkt: 286-287°C

| | | | | | | | | |
|---|---|---|---|---|---|---|---|---|
| Ber.: | C | 47,07 | H | 3,29 | N | 18,18 | S | 10,42 |
| Gef.: | | 47,10 | | 3,28 | | 18,37 | | 10,69 |

Beispiel 17

2-(4-Methoxy-phenyl)-6H-imidazo[1,2-d][1,2,4]triazin-5-on

Hergestellt aus 5-Amino-2H-[1,2,4]triazin-3-on und α-Brom-4-methoxy-acetophenon in Dimethylformamid analog Beispiel 1.
Ausbeute: 42,4 % der Theorie,
Schmelzpunkt: 309-312°C

| | | | | | | |
|---|---|---|---|---|---|---|
| Ber.: | C | 59,50 | H | 4,29 | N | 23,13 |
| Gef.: | | 59,60 | | 4,16 | | 23,59 |

Beispiel 18

2-(4-Methylsulfonamido-phenyl)-8H-imidazo[1,2-a]pyrimidin-7-on

Hergestellt aus 2-Amino-4-hydroxy-pyrimidin und α-Brom-4-methylsulfonamido-acetophenon analog Beispiel 1.
Ausbeute: 31,1 % der Theorie,
Schmelzpunkt: 308-309°C

| | | | | | | | | |
|---|---|---|---|---|---|---|---|---|
| Ber.: | C | 51,31 | H | 3,98 | N | 18,61 | S | 10,54 |
| Gef.: | | 51,00 | | 3,98 | | 18,08 | | 10,65 |

Beispiel 19

2-(2-Methoxy-4-methylsulfonyloxy-phenyl)-6H-imidazo[1,2-c]-pyrimidin-5-on

Hergestellt aus 2-Hydroxy-4-amino-pyrimidin und α-Brom-2-methoxy-4-methylsulfonyloxy-acetophenon analog Beispiel 1.

Ausbeute: 20 % der Theorie,
Schmelzpunkt: 264-266°C

| | | | | | | | | |
|---|---|---|---|---|---|---|---|---|
| Ber.: | C | 50,14 | H | 3,91 | N | 12,53 | S | 9,56 |
| Gef.: | | 49,71 | | 3,95 | | 12,19 | | 9,95 |

Beispiel 20

2-(2,4-Dimethoxy-phenyl)-6H-imidazo[1,2-c]pyrimidin-5-on

Hergestellt aus 2-Hydroxy-4-amino-pyrimidin und α-Brom-2,4-dimethoxy-acetophenon analog Beispiel 1.
Ausbeute: 23 % der Theorie,
Schmelzpunkt: 258-260°C

| | | | | | | |
|---|---|---|---|---|---|---|
| Ber.: | C | 61,98 | H | 5,85 | N | 15,49 |
| Gef.: | | 62,08 | | 5,86 | | 15,24 |

Beispiel 21

2-(4-Methylsulfonamido-phenyl)-5H-imidazo[1,2-b]pyridazin-6-on

Hergestellt aus 2-(4-Methylsulfonamido-phenyl)-6-methylsulfonyloxy-imidazo[1,2-b]pyridazin und Natronlauge analog Beispiel 4.
Ausbeute: 39,2 % der Theorie,
Schmelzpunkt: 315-318°C

| | | | | | | | | |
|---|---|---|---|---|---|---|---|---|
| Ber.: | C | 51,31 | H | 3,97 | N | 18,41 | S | 10,54 |
| Gef.: | | 51,43 | | 4,13 | | 18,20 | | 10,50 |

0185346

## Beispiel 22

2-(4-Amino-phenyl)-5H-imidazo[1,2-b]pyridazin-6-on

Hergestellt aus 2-(4-Acetamido-phenyl)-6-chlor-imidazo-[1,2-b]pyridazin und Kalilauge analog Beispiel 4.
Ausbeute: 95,2 % der Theorie,
Schmelzpunkt: > 330°C
Analyse des Monohydrats:

| | | | | | | |
|---|---|---|---|---|---|---|
| Ber.: | C | 59,02 | H | 4,95 | N | 22,95 |
| Gef.: | | 59,47 | | 4,87 | | 22,63 |

## Beispiel 23

2-(4-Methoxy-2-methylsulfonamido-phenyl)-5H-imidazo[1,2-b]-pyridazin-6-on

Hergestellt aus 6-Chlor-2-(4-methoxy-2-methylsulfonamido-phenyl)-imidazo[1,2-b]pyridazin und Natronlauge analog Beispiel 4.
Ausbeute: 78 % der Theorie,
Schmelzpunkt: 267-276°C

| | | | | | | | | |
|---|---|---|---|---|---|---|---|---|
| Ber.: | C | 50,29 | H | 4,22 | N | 16,76 | S | 9,59 |
| Gef.: | | 49,97 | | 4,10 | | 16,95 | | 9,83 |

## Beispiel 24

2-(2-Methoxy-4-methylsulfonyloxy-phenyl)-5H-imidazo[1,2-b]-pyridazin-6-on

Hergestellt aus 2-(2-Methoxy-4-methylsulfonyloxy-phenyl)-6-methylsulfonyloxy-imidazo[1,2-b]pyridazin und äthanolischer Salzsäure analog Beispiel 4.
Ausbeute: 55 % der Theorie,
Schmelzpunkt: 265-275°C (Zersetzung)

| | | | | | | |
|---|---|---|---|---|---|---|
| Ber.: | C | 48,82 | H | 4,10 | N | 12,20 |
| Gef.: | | 48,27 | | 4,11 | | 11,34 |

Beispiel 25

6-(4-Hydroxy-phenyl)-2-methylmercapto-3H-imidazo[2,1-f]-[1,2,4]triazin-4-on

Hergestellt aus 6-(4-Methoxy-phenyl)-2,4-bis(methylmercapto)-imidazo[2,1-f][1,2,4]triazin und konzentrierter Salzsäure analog Beispiel 5.
Ausbeute: 47,5 % der Theorie,
Schmelzpunkt: 307-309°C

| | | | | | | | | |
|---|---|---|---|---|---|---|---|---|
| Ber.: | C | 52,56 | H | 3,68 | N | 20,43 | S | 11,70 |
| Gef.: | | 52,26 | | 3,78 | | 20,66 | | 11,73 |

Beispiel 26

7-(4-Methylsulfonyloxy-phenyl)-1H-imidazo[1,2-a][1.3.5]triazin-2-on

Hergestellt aus 2-Amino-4-hydroxy-1,3,5-triazin und α-Brom-4-methylsulfonyloxy-acetophenon analog Beispiel 1.
Ausbeute: 29,8 % der Theorie,
Schmelzpunkt: 234-236°C

| | | | | | | | | |
|---|---|---|---|---|---|---|---|---|
| Ber.: | C | 47,05 | H | 3,29 | N | 18,29 | S | 10,47 |
| Gef.: | | 47,03 | | 3,27 | | 18,66 | | 10,49 |

Beispiel 27

7-(4-Dimethylaminosulfonyl-phenyl)-1H-imidazo[1,2-a][1.3.5]-triazin-2-on

Hergestellt aus 2-Amino-4-hydroxy-1,3,5-triazin und α-Brom-4-dimethylaminosulfonyl-acetophenon analog Beispiel 1.
Ausbeute: 39,1 % der Theorie,
Schmelzpunkt: 283-285°C

| | | | | | | | | |
|---|---|---|---|---|---|---|---|---|
| Ber.: | C | 48,89 | H | 4,10 | N | 21,93 | S | 10,04 |
| Gef.: | | 49,32 | | 4,30 | | 21,74 | | 10,23 |

Beispiel 28

2-(2-Methoxy-4-methylsulfonamido-phenyl)-8H-imidazo[1,2-a]-pyrimidin-7-on

Hergestellt aus 2-Amino-4-hydroxy-pyrimidin und α-Brom-2-methoxy-4-methylsulfonamido-acetophenon analog Beispiel 1.
Ausbeute: 36,6 % der Theorie,
Schmelzpunkt: 314-316°C
Analyse des Monohydrats:

| | | | | | | | | |
|---|---|---|---|---|---|---|---|---|
| Ber.: | C | 47,71 | H | 4,57 | N | 15,90 | S | 9,09 |
| Gef.: | | 47,80 | | 4,37 | | 15,89 | | 9,34 |

Beispiel 29

2-(2-Methoxy-4-methylsulfonyl-phenyl)-7H-imidazo[1,2-a]-pyrazin-8-on

Hergestellt aus 3-Amino-1H-pyrazin-2-on und α-Brom-2-methoxy-4-methylsulfonyl-acetophenon analog Beispiel 1.

Ausbeute: 56 % der Theorie,
Schmelzpunkt: 283-286°C (Zersetzung)
UV-Spektrum (Ethanol): 268 nm (0,56)
308 nm (0,48)
320 nm (0,45)

Beispiel 30

7-(2-Methoxy-4-methylsulfonyl-phenyl)-1H,3H-imidazo[1,2-a]-[1.3.5]triazin-2,4-dion

Hergestellt aus 6-Amino-1H,3H-[1.3.5]triazin-2,4-dion und α-Brom-2-methoxy-4-methylsulfonyl-acetophenon analog Beispiel 1.
Ausbeute: 22 % der Theorie,
Schmelzpunkt: > 300°C
Massenspektrum: $M^+$: 336 m/e
UV-Spektrum (Ethanol + NaOH): 245 nm (0,46)
330 nm (0,28)

Beispiel 31

7-(2-Methoxy-4-methylsulfonyloxy-phenyl)-1H,3H-imidazo-[1,2-a][1.3.5]triazin-2,4-dion

Hergestellt aus 6-Amino-1H,3H-[1.3.5]triazin-2,4-dion und α-Brom-2-methoxy-4-methylsulfonyloxy-acetophenon analog Beispiel 1.
Ausbeute: 22 % der Theorie,
Schmelzpunkt: > 300°C

| | | | | | | |
|---|---|---|---|---|---|---|
| Ber.: | C | 44,32 | H | 3,43 | N | 15,90 |
| Gef.: | | 44,51 | | 3,55 | | 15,78 |

Massenspektrum: $M^+$: 352 m/e
UV-Spektrum (Ethanol): 290-304 (0,09)

Beispiel 32

2-(2-Methoxy-4-methylsulfonyloxy-phenyl)-7H-imidazo[1.2-a]-pyrazin-8-on

Hergestellt aus 3-Amino-1H-pyrazin-2-on und α-Brom-2-methoxy-4-methylsulfonyloxy-acetophenon analog Beispiel 1.
Ausbeute: 43 % der Theorie,
Schmelzpunkt: 273-275°C

| | | | | | | | | |
|---|---|---|---|---|---|---|---|---|
| Ber.: | C | 50,11 | H | 3,91 | N | 12,52 | S | 9,55 |
| Gef.: | | 49,90 | | 3,99 | | 12,82 | | 9,61 |

Beispiel 33

2-(2-Methoxy-4-methylsulfonamido-phenyl)-6H-imidazo[1,2-c]-pyrimidin-5-on

Hergestellt aus 2-Hydroxy-4-amino-pyrimidin und α-Brom-2-methoxy-4-methylsulfonamido-acetophenon analog Beispiel 1.
Ausbeute: 28,4 % der Theorie,
Schmelzpunkt: 240-243°C

| | | | | | | | | |
|---|---|---|---|---|---|---|---|---|
| Ber.: | C | 50,29 | H | 4,22 | N | 16,76 | S | 9,59 |
| Gef.: | | 49,90 | | 4,31 | | 16,54 | | 9,65 |

Beispiel 34

7-(2-Methoxy-4-methylsulfonamido-phenyl)-1H-imidazo[1,2-a]-[1.3.5]triazin-2-on

Hergestellt aus 2-Amino-6-hydroxy-[1.3.5]triazin und α-Brom-2-methoxy-4-methylsulfonamido-acetohenon analog Beispiel 1.
Ausbeute: 25,4 % der Theorie,

Schmelzpunkt: 245-248°C

| | | | | | | | | |
|---|---|---|---|---|---|---|---|---|
| Ber.: | C | 46,56 | H | 3,91 | N | 20,09 | S | 9,56 |
| Gef.: | | 46,31 | | 4,03 | | 20,38 | | 9,37 |

Beispiel 35

2-(2-Methoxy-4-methylsulfonyloxy-phenyl)-8H-imidazo[1,2-a]-pyrimidin-7-on

Hergestellt aus 2-Amino-4-hydroxy-pyrimidin und α-Brom-2-methoxy-4-methylsulfonyloxy-acetophenon analog Beispiel 1.
Ausbeute: 24 % der Theorie,
Schmelzpunkt: 253-255°C

| | | | | | | | | |
|---|---|---|---|---|---|---|---|---|
| Ber.: | C | 50,14 | H | 3,90 | N | 12,53 | S | 9,56 |
| Gef.: | | 50,30 | | 3,73 | | 12,33 | | 9,44 |

Beispiel 36

2-(2-Methoxy-4-methylaminosulfonyl-phenyl)-6H-imidazo[1,2-c]-pyrimidin-5-on

Hergestellt aus 2-Hydroxy-4-amino-pyrimidin und α-Brom-2-methoxy-4-methylaminosulfonyl-acetophenon analog Beispiel 1.
Ausbeute: 37 % der Theorie,
Schmelzpunkt: > 300°C

| | | | | | | | | |
|---|---|---|---|---|---|---|---|---|
| Ber.: | C | 47,91 | H | 4,57 | N | 15,90 | S | 9,09 |
| Gef.: | | 48,00 | | 4,43 | | 15,79 | | 9,27 |

Beispiel 37

2-(2-Methoxy-4-methylsulfonamido-phenyl)-6H-imidazo[1,2-c]-pyrimidin-5-on

Hergestellt aus 2-Hydroxy-4-amino-pyrimidin und α-Brom-2-methoxy-4-methylsulfonamido-acetophenon analog Beispiel 1.
Ausbeute: 22,7 % der Theorie,
Schmelzpunkt: > 300°C

| | | | | | | | | |
|---|---|---|---|---|---|---|---|---|
| Ber.: | C | 47,71 | H | 4,57 | N | 15,90 | S | 9,09 |
| Gef.: | | 47,45 | | 4,46 | | 15,75 | | 8,92 |

Beispiel 38

7-(2-Methoxy-4-methylaminosulfonyl-phenyl)-1H-imidazo[1,2-a]-[1,3,5]triazin-2-on

Hergestellt aus Azacytosin und α-Brom-2-methoxy-4-methylaminosulfonyl-acetophenon analog Beispiel 1.
Ausbeute: 43,7 % der Theorie,
Schmelzpunkt: > 300°C

| | | | | | | | | |
|---|---|---|---|---|---|---|---|---|
| Ber.: | C | 44,18 | H | 4,28 | N | 19,82 | S | 9,07 |
| Gef.: | | 43,91 | | 4,42 | | 19,46 | | 9,12 |

Beispiel 39

7-(2-Methoxy-4-methylsulfonamido-phenyl)-1H-imidazo[1,2-a]-[1,3,5]-triazin-2-on

Hergestellt aus Azacytosin und α-Brom-2-methoxy-4-methylsulfonamido-acetophenon analog Beispiel 1.

Ausbeute: 40,6 % der Theorie,
Schmelzpunkt: > 300°C

| | | | | | | | | |
|---|---|---|---|---|---|---|---|---|
| Ber.: | C | 44,18 | H | 4,28 | N | 19,82 | S | 9,07 |
| Gef.: | | 43,99 | | 4,35 | | 20,02 | | 9,02 |

Beispiel 40

2-(2-Methoxy-4-cyano-phenyl)-8H-imidazo[1,2-a]pyrimidin-7-on

Hergestellt aus 2-Amino-4-hydroxy-pyrimidin und α-Brom-2-methoxy-4-cyano-acetophenon analog Beispiel 1.
Ausbeute: 42,1 % der Theorie,
Schmelzpunkt: 308-310°C

| | | | | | | |
|---|---|---|---|---|---|---|
| Ber.: | C | 61,74 | H | 4,03 | N | 20,37 |
| Gef.: | | 62,01 | | 4,03 | | 20,03 |

Beispiel 41

2-(2-Methoxy-4-dimethylaminocarbonyl-phenyl)-6H-imidazo-[1,2-c]pyrimidin-5-on

Hergestellt aus 2-Hydroxy-4-amino-pyrimidin und α-Brom-2-methoxy-4-dimethylaminocarbonyl-acetophenon analog Beispiel 1.
Ausbeute: 21,6 % der Theorie,
Schmelzpunkt: 245-250°C

| | | | | | | |
|---|---|---|---|---|---|---|
| Ber.: | C | 61,53 | H | 5,16 | N | 17,94 |
| Gef.: | | 61,26 | | 5,19 | | 17,98 |

Beispiel 42

2-(2-Methoxy-4-methylaminocarbonyl-phenyl)-8H-imidazo[1,2-a]-pyrimidin-7-on

Hergestellt aus 2-Amino-4-hydroxy-pyrimidin und α-Brom-2-methoxy-methylaminocarbonyl-acetophenon analog Beispiel 1.
Ausbeute: 41,6 % der Theorie,
Schmelzpunkt des Hydrats: 272-274°C

| | | | | | | |
|---|---|---|---|---|---|---|
| Ber.: | C | 56,96 | H | 5,10 | N | 17,71 |
| Gef.: | | 56,70 | | 4,99 | | 18,01 |

Beispiel 43

2-(2-Methoxy-4-methylaminocarbonyl-phenyl)-6H-imidazo[1,2-c]-pyrimidin-5-on

Hergestellt aus 2-Hydroxy-4-amino-pyrimidin und α-Brom-2-methoxy-4-methylaminocarbonyl-acetophenon analog Beispiel 1.
Ausbeute: 39,2 % der Theorie,
Schmelzpunkt: 277-279°C

| | | | | | | |
|---|---|---|---|---|---|---|
| Ber.: | C | 58,62 | H | 4,92 | N | 18,23 |
| Gef.: | | 58,58 | | 4,95 | | 18,25 |

Beispiel 44

2-(2-Methoxy-4-dimethylaminocarbonyl-phenyl)-8H-imidazo-[1,2-a]pyrimidin-7-on

Hergestellt aus 2-Amino-4-hydroxy-pyrimidin und α-Brom-2-methoxy-4-dimethylaminocarbonyl-acetophenon analog Beispiel 1.

Ausbeute: 38,1 % der Theorie,
Schmelzpunkt: 248-251°C

| | | | | | | |
|---|---|---|---|---|---|---|
| Ber.: | C | 61,53 | H | 5,16 | N | 17,94 |
| Gef.: | | 61,65 | | 5,17 | | 17,81 |

Beispiel 45

2-(2,4-Dimethoxy-5-dimethylaminosulfonyl-phenyl)-8H-imidazo-[1,2-a]pyrimidin-7-on

Hergestellt aus 2-(2,4-Dimethoxy-phenyl)-8H-imidazo[1,2-a]-pyrimidin-7-on und Chlorsulfonsäure und anschließende Umsetzung mit 40%igem Dimethylamin analog Beispiel 3.
Ausbeute: 45 % der Theorie,
Schmelzpunkt: 300-303°C

| | | | | | | | | |
|---|---|---|---|---|---|---|---|---|
| Ber.: | C | 48,47 | H | 5,08 | N | 14,90 | S | 8,62 |
| Gef.: | | 48,41 | | 5,29 | | 14,90 | | 8,62 |

Beispiel 46

7-(2-Methoxy-4-dimethylaminosulfonyl-phenyl)-1H-imidazo-[1,2-a][1,3,5]triazin-2-on

Hergestellt aus Azacytosin und α-Brom-2-methoxy-4-dimethylaminosulfonyl-acetophenon analog Beispiel 1.
Ausbeute: 28 % der Theorie,
Schmelzpunkt: 285-287°C

| | | | | | | | | |
|---|---|---|---|---|---|---|---|---|
| Ber.: | C | 45,76 | H | 4,66 | N | 19,06 | S | 9,18 |
| Gef.: | | 45,52 | | 4,68 | | 18,91 | | 9,67 |

Beispiel 47

2-(2-Methoxy-4-sulfamyl-phenyl)-8H-imidazo[1,2-a]-pyrimidin-7-on

Hergestellt aus 2-Amino-4-hydroxy-pyrimidin und α-Brom-2-methoxy-4-sulfamyl-acetophenon analog Beispiel 1.
Ausbeute: 43,7 % der Theorie,
Schmelzpunkt: 284-286°C

| | | | | | | | | |
|---|---|---|---|---|---|---|---|---|
| Ber.: | C | 48,74 | H | 3,78 | N | 17,49 | S | 10,01 |
| Gef.: | | 48,69 | | 3,87 | | 17,30 | | 10,17 |

Beispiel 48

2-(2-Methoxy-4-sulfamyl-phenyl)-6H-imidazo[1,2-c]-pyrimidin-5-on

Hergestellt aus 2-Hydroxy-4-amino-pyrimidin und α-Brom-2-methoxy-4-sulfamyl-acetophenon analog Beispiel 1.
Ausbeute: 39,4 % der Theorie,
Schmelzpunkt: 284-286°C

| | | | | | | | | |
|---|---|---|---|---|---|---|---|---|
| Ber.: | C | 48,74 | H | 3,78 | N | 17,49 | S | 10,01 |
| Gef.: | | 48,59 | | 3,97 | | 17,40 | | 10,21 |

Beispiel 49

2-(2-Methoxy-4-methylaminosulfonyl-phenyl)-8H-imidazo[1,2-a]-pyrimidin-7-on

Hergestellt aus 2-Amino-4-hydroxy-pyrimidin und α-Brom-2-methoxy-4-methylaminosulfonyl-acetophenon analog Beispiel 1.

Ausbeute: 22 % der Theorie,

Schmelzpunkt: 300-302°C

| | | | | | | | | |
|---|---|---|---|---|---|---|---|---|
| Ber.: | C | 50,28 | H | 4,22 | N | 16,76 | S | 9,59 |
| Gef.: | | 50,15 | | 4,39 | | 16,65 | | 9,68 |

## Beispiel 50

2-(2-Methoxy-4-aminocarbonyl-phenyl)-8H-imidazo[1,2-a]pyridin-7-on-hydrat

Hergestellt aus 2-Amino-4-hydroxy-pyrimidin und α-Brom-2-methoxy-4-aminocarbonyl-acetophenon analog Beispiel 1.

Ausbeute: 35 % der Theorie,

Schmelzpunkt: > 330°C

| | | | | | | |
|---|---|---|---|---|---|---|
| Ber.: | C | 55,63 | H | 4,67 | N | 18,53 |
| Gef.: | | 55,38 | | 4,58 | | 18,29 |

## Beispiel 51

7-(2-Methoxy-4-cyano-phenyl)-1H-imidazo[1,2-a][1,3,5]triazin-2-on

Hergestellt aus Azacytosin und α-Brom-2-methoxy-4-cyano-acetophenon analog Beispiel 1.

Ausbeute: 36,8 % der Theorie,

Schmelzpunkt: 270-272°C

| | | | | | | |
|---|---|---|---|---|---|---|
| Ber.: | C | 51,48 | H | 3,98 | N | 23,09 |
| Gef.: | | 51,80 | | 3,98 | | 22,80 |

Beispiel 52

2-(2,4-Dimethoxy-phenyl)-8H-imidazo[1,2-a]pyrimidin-7-on

Hergestellt aus 2-Amino-4-hydroxy-pyrimidin und α-Brom-2,4-dimethoxy-acetophenon analog Beispiel 1.
Ausbeute: 55,8 % der Theorie,
Schmelzpunkt: 270-273°C

| | | | | | | |
|---|---|---|---|---|---|---|
| Ber.: | C | 61,99 | H | 4,83 | N | 15,49 |
| Gef.: | | 61,68 | | 4,91 | | 15,46 |

Beispiel 53

2-(2-Methoxy-4-aminocarbonyl-phenyl)-6H-imidazo[1,2-c]pyrimidin-5-on

Hergestellt aus 2-Hydroxy-4-amino-pyrimidin und α-Brom-2-methoxy-4-aminocarbonyl-acetophenon analog Beispiel 1.
Ausbeute: 30 % der Theorie,
Schmelzpunkt: 240-245°C

| | | | | | | |
|---|---|---|---|---|---|---|
| Ber.: | C | 59,15 | H | 4,26 | N | 19,71 |
| Gef.: | | 59,39 | | 4,21 | | 19,62 |

Beispiel 54

2-(2-Methoxy-4-dimethylaminosulfonyl-phenyl)-6H-imidazo-[1,2-c]pyrimidin-5-on

Hergestellt aus 2-Hydroxy-4-amino-pyrimidin und α-Brom-2-methoxy-4-dimethylaminosulfonyl-acetophenon analog Beispiel 1.

Ausbeute: 30,6 % der Theorie,
Schmelzpunkt: 219-221°C

| | | | | | | | | |
|---|---|---|---|---|---|---|---|---|
| Ber.: | C | 49,71 | H | 4,98 | N | 15,37 | S | 8,97 |
| Gef.: | | 49,69 | | 5,01 | | 15,27 | | 9,03 |

Beispiel 55

2-(2-Methoxy-4-cyano-phenyl)-6H-imidazo[1,2-c]pyrimidin-5-on

Hergestellt aus 2-Hydroxy-4-amino-pyrimidin und α-Brom-2-methoxy-4-cyano-acetophenon analog Beispiel 1.
Ausbeute: 27 % der Theorie,
Schmelzpunkt: 308-310°C

| | | | | | | |
|---|---|---|---|---|---|---|
| Ber.: | C | 61,08 | H | 4,02 | N | 20,35 |
| Gef.: | | 61,15 | | 3,98 | | 19,97 |

Beispiel 56

2-(2-Methoxy-4-dimethylaminosulfonyl-phenyl)-8H-imidazo-[1,2-a]pyrimidin-7-on

Hergestellt aus 2-Amino-4-hydroxy-pyrimidin und α-Brom-2-methoxy-4-dimethylaminosulfonyl-acetophenon analog Beispiel 1.
Ausbeute: 39,4 % der Theorie,
Schmelzpunkt: 297-300°C

| | | | | | | | | |
|---|---|---|---|---|---|---|---|---|
| Ber.: | C | 51,86 | H | 4,35 | N | 16,13 | S | 9,23 |
| Gef.: | | 51,58 | | 4,54 | | 16,10 | | 9,15 |

Beispiel 57

7-(2-Methoxy-4-methylsulfonyloxy-phenyl)-1H-imidazo[1,2-a]-[1,3,5]triazin-2-on

Hergestellt aus 2-Amino-4-hydroxy-[1,3,5]triazin und α-Brom-2-methoxy-4-methylsulfonyloxy-acetophenon analog Beispiel 1.
Ausbeute: 17,5 % der Theorie,
Schmelzpunkt: 258-261°C

| | | | | | | | | |
|---|---|---|---|---|---|---|---|---|
| Ber.: | C | 44,06 | H | 3,98 | N | 15,81 | S | 9,14 |
| Gef.: | | 43,86 | | 3,93 | | 15,64 | | 9,21 |

Beispiel 58

7-(2-Methoxy-4-methylsulfonyl-phenyl)-1H,3H-imidazo[1,2-a]-[1,3,5]triazin-2,4-dion

Hergestellt aus 6-Amino-1H,3H-[1,3,5]triazin-2,4-dion und α-Brom-2-methoxy-4-methylsulfonyl-acetophenon analog Beispiel 1.
Ausbeute: 22 % der Theorie,
Schmelzpunkt: > 300°C

| | | | | | | |
|---|---|---|---|---|---|---|
| Ber.: | C | 46,42 | H | 3,59 | N | 16,66 |
| Gef.: | | 46,38 | | 3,71 | | 16,78 |

Massenspektrum: $M^+$ = 336 m/e.

Beispiel 59

7-(2-Methoxy-4-methylsulfonyloxy-phenyl)-1H,3H-imidazo-[1,2-a][1,3,5]triazin-2,4-dion

Hergestellt aus 6-Amino-1H,3H-[1,3,5]triazin-2,4-dion und

 

α-Brom-2-methoxy-4-methylsulfonyloxy-acetophenon analog Beispiel 1.
Ausbeute: 22 % der Theorie,
Schmelzpunkt: > 300°C

| | | | | | | |
|---|---|---|---|---|---|---|
| Ber.: | C | 44,32 | H | 3,43 | N | 15,90 |
| Gef.: | | 44,51 | | 3,55 | | 15,68 |

Massenspektrum: $M^+$ = 352 m/e.

Beispiel 60

7-(2-Methoxy-4-dimethylaminosulfonyl-phenyl)-1H,3H-imidazo-[1,2-a][1,3,5]triazin-2,4-dion

Hergestellt aus 6-Amino-1H,3H-[1,3,5]triazin-2,4-dion und α-Brom-2-methoxy-4-dimethylaminosulfonyl-acetophenon analog Beispiel 1.
Ausbeute: 12 % der Theorie,
Schmelzpunkt: > 300°C
$R_f$-Wert: 0,6 (Kieselgel, Laufmittel: Methylenchlorid/-Methanol = 10:2)
UV-Spektrum (Ethanol): 300-320 nm (0,075)

Beispiel 61

2-(2-Methoxy-4-methylsulfonyl-phenyl)-7H-imidazo[1,2-a]-pyrazin-8-on

Hergestellt aus 3-Amino-1H-pyrazin-2-on und α-Brom-2-methoxy-4-methylsulfonyl-acetophenon analog Beispiel 1.
Ausbeute: 56 % der Theorie,
Schmelzpunkt: 283-286°C (Zers.).

Beispiel 62

2-(2-Methoxy-4-methylsulfonyloxy-phenyl)-7H-imidazo[1,2-a]-pyrazin-8-on

Hergestellt aus 3-Amino-1H-pyrazin-2-on und α-Brom-2-methoxy-4-methylsulfonyloxy-acetophenon analog Beispiel 1.
Ausbeute: 43 % der Theorie,
Schmelzpunkt: 273-275°C

| | | | | | | | | |
|---|---|---|---|---|---|---|---|---|
| Ber.: | C | 50,11 | H | 3,91 | N | 12,52 | S | 9,55 |
| Gef.: | | 49,80 | | 3,99 | | 12,82 | | 9,61 |

Beispiel 63

2-(2-Naphthyl)-7H-imidazo[1,2-a]pyrazin-8-on

Hergestellt aus 3-Amino-1H-pyrazin-2-on und 2-(2-Bromacetyl)-naphthalin analog Beispiel 1.
Ausbeute: 47 % der Theorie,
Schmelzpunkt: > 300°C

| | | | | | | |
|---|---|---|---|---|---|---|
| Ber.: | C | 75,55 | H | 4,24 | N | 16,08 |
| Gef.: | | 75,17 | | 4,14 | | 15,83 |

Beispiel 64

6-(4-Hydroxy-2-methoxy-phenyl)-2-methylmercapto-1H-imidazo-[2,1-f][1,2,4]triazin-4-on

Hergestellt aus 6-(2-Methoxy-4-hydroxy-phenyl)-2,4-bis-methylmercapto-imidazo[2,1-f][1,2,4]triazin und konzentrierter Salzsäure analog Beispiel 4.

Ausbeute: 28,1 % der Theorie,
Schmelzpunkt: 307-309°C

| | | | | | | | | |
|---|---|---|---|---|---|---|---|---|
| Ber.: | C | 51,32 | H | 3,98 | N | 18,41 | S | 10,52 |
| Gef.: | | 51,21 | | 4,10 | | 18,15 | | 10,85 |

Beispiel 65

2-(1-Methoxy-naphth-4-yl)-8H-imidazo[1,2-a]pyrimidin-7-on

Hergestellt aus α-Brom-4-(1-methoxy)-acetonaphthon und 2-Amino-4-hydroxy-pyrimidin analog Beispiel 1.
Ausbeute: 15 % der Theorie,
Schmelzpunkt: 300-301°C (Zers.)

| | | | | | | |
|---|---|---|---|---|---|---|
| Ber.: | C | 70,09 | H | 4,50 | N | 14,42 |
| Gef.: | | 69,87 | | 4,49 | | 14,31 |

Beispiel 66

2-(2-Methoxy-naphth-6-yl)-6H-imidazo[1,2-c]pyrimidin-5-on

Hergestellt aus α-Brom-6-(2-methoxy)-acetonaphthon und 2-Hydroxy-4-amino-pyrimidin analog Beispiel 1.
Ausbeute: 15,3 % der Theorie,
Schmelzpunkt: > 300°C

| | | | | | | |
|---|---|---|---|---|---|---|
| Ber.: | C | 70,09 | H | 4,50 | N | 14,42 |
| Gef.: | | 70,29 | | 4,62 | | 14,54 |

Beispiel 67

2-(1-Methoxy-naphth-2-yl)-8H-imidazo[1,2-a]pyrimidin-7-on

Hergestellt aus 1-Brom-2-(1-methoxy)-acetonaphthon und

2-Amino-4-hydroxy-pyrimidin analog Beispiel 1.
Ausbeute: 3,1 % der Theorie,
Schmelzpunkt: 215-216°C

| | | | | | | |
|---|---|---|---|---|---|---|
| Ber.: | C | 70,09 | H | 4,50 | N | 14,43 |
| Gef.: | | 70,06 | | 4,39 | | 14,11 |

## Beispiel 68

### 2-(Naphth-2-yl)-8H-imidazo[1,2-a]pyrimidin-7-on

Hergestellt aus α-Brom-2-acetonaphthon und 2-Amino-4-hydroxy-pyrimidin analog Beispiel 1.
Ausbeute: 11 % der Theorie,
Schmelzpunkt: > 250°C

| | | | | | | |
|---|---|---|---|---|---|---|
| Ber.: | C | 73,55 | H | 4,24 | N | 16,08 |
| Gef.: | | 73,26 | | 4,30 | | 15,82 |

## Beispiel 69

### 2-(4-Amino-2-methoxy-phenyl)-5H-imidazo[1,2-b]pyridazin-6-on

1,7 g (5,36 mMol) 2-(4-Acetamino-2-methoxy-phenyl)-6-chlor-imidazo[1,2-b]pyridazin werden 32 Stunden in 150 ml 4n Kalilauge zum Rückfluß erhitzt. Nach Abkühlen und Filtration wird mit Eisessig auf pH 6,5 gestellt. Der ausgefallene Niederschlag wird abgesaugt, mit Wasser gewaschen und im Vakuumtrockenschrank über Phosphorpentoxid getrocknet.
Ausbeute: 84 % der Theorie,
Schmelzpunkt: 243-250°C (Zers.)

| | | | | | | |
|---|---|---|---|---|---|---|
| Ber.: | C | 60,93 | H | 4,72 | N | 21,86 |
| Gef.: | | 60,76 | | 4,67 | | 20,50 |

Beispiel 70

2-(2-Methoxy-4-sulfamyl-phenyl)-5H-imidazo[1,2-b]pyridazin-6-on

Hergestellt aus 6-Chlor-2-(2-methoxy-4-sulfamyl-phenyl)-imidazo[1,2-b]pyridazin und 4n Kalilauge analog Beispiel 4.
Ausbeute: 38 % der Theorie,
Schmelzpunkt: 297-300°C (Zers.)

| | | | | | | | | |
|---|---|---|---|---|---|---|---|---|
| Ber.: | C | 46,15 | H | 4,17 | N | 16,56 | S | 9,48 |
| Gef.: | | 46,36 | | 4,09 | | 16,70 | | 9,99 |

Massenspektrum: $M^+$ = 320 m/e.

Beispiel 71

2-(2-Methoxy-4-methylsulfonamido-phenyl)-5H-imidazo[1,2-b]-pyridazin-6-on

Hergestellt aus 6-Chlor-2-(2-methoxy-4-methylsulfonamido-phenyl)-imidazo[1,2-b]pyridazin und 4n Kalilauge analog Beispiel 4.
Ausbeute: 64 % der Theorie,
Schmelzpunkt: 303-305°C (Zers.)

| | | | | | | | | |
|---|---|---|---|---|---|---|---|---|
| Ber.: | C | 50,29 | H | 4,22 | N | 16,76 | S | 9,59 |
| Gef.: | | 50,29 | | 4,09 | | 16,81 | | 9,81 |

Beispiel 72

2-(2-Methoxy-4-methylsulfonyloxy-phenyl)-6H-imidazo[1,2-d]-[1,2,4]triazin-5-on

336 mg (3 mMol) 5-Amino-2H-1,2,4-triazin-3-on werden in

20 ml absolutem Dimethylformamid suspendiert, anschließend gibt man 1,14 g (3,5 mMol) ω-Brom-2-methoxy-4-methylsulfonyloxy-acetophenon zu. Anschließend wird 2 Stunden auf 120°C unter Rühren erhitzt, wobei die gelbe Suspension in eine klare orangerote Lösung übergeht. Die Reaktionslösung wird in 100 ml Eiswasser eingerührt, wobei das anfangs viskose Öl langsam durchkristallisiert. Das kristalline Rohprodukt wird abgesaugt und danach noch einmal aus Äthanol/Dimethylformamid (3:1) unter Zusatz von Aktivkohle umkristallisiert.

Ausbeute: 145 mg (14,3 % der Theorie),

Schmelzpunkt: 262-264°C (Zers.)

| | | | | | | | | |
|---|---|---|---|---|---|---|---|---|
| Ber.: | C | 46,44 | H | 3,60 | N | 16,66 | S | 9,54 |
| Gef.: | | 46,45 | | 3,72 | | 16,70 | | 9,50 |

## Beispiel 73

### 2-(4-Dimethylaminosulfonyl-2-methoxy-phenyl)-5H-imidazo-[1,2-b]pyridazin-6-on

Hergestellt aus 6-Chlor-2-(4-dimethylaminosulfonyl-2-methoxy-phenyl)-imidazo[1,2-b]pyridazin und 4n Kalilauge analog Beispiel 4.

Ausbeute: 49 % der Theorie,

Schmelzpunkt: 285-287°C (Zers.)

| | | | | | | | | |
|---|---|---|---|---|---|---|---|---|
| Ber.: | C | 51,71 | H | 4,63 | N | 16,08 | S | 9,20 |
| Gef.: | | 51,48 | | 4,71 | | 16,17 | | 9,44 |

Analog den vorstehenden Beispielen werden folgende Verbindungen erhalten:

2-(4-Aminocarbonyl-phenyl)-6H-imidazo[1,2-c]pyrimidin-5-on

7-(4-Methylaminocarbonyl-phenyl)-1H-imidazo[1,2-a][1,3,5]-triazin-2-on

2-(2-Methoxy-4-methylsulfonyl-phenyl)-6H-imidazo[1,2-c]pyrimidin-5-on

2-(2,4-Dimethoxy-5-dimethylaminosulfonyl-phenyl)-6H-imidazo-[1,2-c]pyrimidin-5-on

2-(2-Methoxy-4-methylsulfonyloxy-phenyl)-8H-imidazo[1,2-c]-pyrimidin-7-on

2-(2-Methoxy-4-methylsulfonyloxy-phenyl)-5H-imidazo[1,2-b]-pyridazin-6-on

2-(4-Methylsulfonamido-phenyl)-5H-imidazo[1,2-b]pyridazin-6-on

2-(4-Amino-phenyl)-5H-imidazo[1,2-b]pyridazin-6-on

6-(2-Methoxy-4-methylsulfonyloxy-phenyl)-1H-imidazo[2,1-f]-[1,2,4]triazin-2-on

6-(4-Methylsulfonyloxy-phenyl)-1H-imidazo[2,1-f][1,2,4]triazin-2-on

6-(2-Methoxy-4-sulfamyl-phenyl)-1H-imidazo[2,1-f][1,2,4]-triazin-2-on

6-(2-Methoxy-4-methylaminosulfonyl-phenyl)-1H-imidazo[2,1-f]-[1,2,4]triazin-2-on

6-(2-Methoxy-4-dimethylaminosulfonyl-phenyl)-1H-imidazo-[2,1-f][1,2,4]triazin-2-on

6-(2-Methoxy-4-methylmercapto-phenyl)-1H-imidazo[2,1-f]-[1,2,4]triazin-2-on

6-(2-Methoxy-4-methylsulfinyl-phenyl)-1H-imidazo[2,1-f]-[1,2,4]triazin-2-on

6-(2-Methoxy-4-methylsulfonyl-phenyl)-1H-imidazo[2,1-f]-[1,2,4]triazin-2-on

6-(4-Benzyloxy-2-methoxy-phenyl)-2-methylmercapto-3H-imidazo-[2,1-f][1,2,4]triazin-4-on

6-(4-Benzyloxy-2-methoxy-phenyl)-1H,3H-imidazo[2,1-f][1,2,4]-triazin-2,4-dion

6-(4-Hydroxy-2-methoxy-phenyl)-3H-imidazo[2,1-f][1,2,4]-triazin-4-on

6-(2-Methoxy-4-methylsulfonyloxy-phenyl)-3H-imidazo[2,1-f]-[1,2,4]triazin-4-on

6-(4-Hydroxy-2-methoxy-phenyl)-1H,3H-imidazo[2,1-f][1,2,4]-triazin-2,4-dion

6-(2-Methoxy-4-methylsulfonyloxy-phenyl)-1H,3H-imidazo[2,1-f]-[1,2,4]triazin-2,4-dion

2-(2-Methoxy-4-methylsulfonyloxy-phenyl)-7H-imidazo[1,2-d]-[1,2,4]triazin-8-on

2-(4-Methylsulfonyloxy-phenyl)-7H-imidazo[1,2-d][1,2,4]triazin-8-on

2-(2-Methoxy-4-sulfamyl-phenyl)-7H-imidazo[1,2-d][1,2,4]-triazin-8-on

2-(2-Methoxy-4-methylaminosulfonyl-phenyl)-7H-imidazo-[1,2-d][1,2,4]triazin-8-on

2-(2-Methoxy-4-dimethylaminosulfonyl-phenyl)-7H-imidazo-[1,2-d][1,2,4]triazin-8-on

2-(2-Methoxy-4-methylmercapto-phenyl)-7H-imidazo[1,2-d]-[1,2,4]triazin-8-on

2-(2-Methoxy-4-methylsulfinyl-phenyl)-7H-imidazo[1,2-d]-[1,2,4]triazin-8-on

2-(2-Methoxy-4-methylsulfonyl-phenyl)-7H-imidazo[1,2-d]-[1,2,4]triazin-8-on

2-(2-Methoxy-4-sulfamyl-phenyl)-6H-imidazo[1,2-d][1,2,4]-triazin-5-on

2-(2-Methoxy-4-methylaminosulfonyl-phenyl)-6H-imidazo-[1,2-d][1,2,4]triazin-5-on

2-(2-Methoxy-4-dimethylaminosulfonyl-phenyl)-6H-imidazo-[1,2-d][1,2,4]triazin-5-on

2-(2-Methoxy-4-methylmercapto-phenyl]-6H-imidazo[1,2-d]-[1,2,4]triazin-5-on

2-(2-Methoxy-4-methylsulfinyl-phenyl)-6H-imidazo[1,2-d]-[1,2,4]triazin-5-on

2-(2-Methoxy-4-methylsulfonyl-phenyl)-6H-imidazo[1,2-d]-[1,2,4]triazin-5-on

2-(2-Methoxy-4-methylsulfonamido-phenyl)-7H-imidazo[1,2-a]-pyrazin-8-on

2-(2-Methoxy-4-dimethylaminosulfonyl-phenyl)-7H-imidazo-[1,2-a]pyrazin-8-on

2-(2-Methoxy-4-methylsulfonyloxy-phenyl)-6H,8H-imidazo[1,2-a]-pyrimidin-5,7-dion

2-(2-Methoxy-4-methylsulfonyl-phenyl)-6H,8H-imidazo[1,2-a]-pyrimidin-5,7-dion

2-(2-Methoxy-4-methylsulfonamido-phenyl)-6H,8H-imidazo[1,2-a]-pyrimidin-5,7-dion

2-(2-Methoxy-4-dimethylaminosulfonyl-phenyl)-6H,8H-imidazo-[1,2-a]pyrimidin-5,7-dion

6-(2-Methoxy-4-methylsulfonyloxy-phenyl)-1H,3H-imidazo-[2,1-f][1,2,4]triazin-2,4-dion

6-(2-Methoxy-4-methylsulfonyl-phenyl)-1H,3H-imidazo[2,1-f]-[1,2,4]triazin-2,4-dion

6-(2-Methoxy-4-methylsulfonamido-phenyl)-1H,3H-imidazo[2,1-f]-[1,2,4]triazin-2,4-dion

6-(2-Methoxy-4-dimethylaminosulfonyl-phenyl)-1H,3H-imidazo-[2,1-f][1,2,4]triazin-2,4-dion

7-(2-Methoxy-4-methylsulfonamido-phenyl)-1H,3H-imidazo[1,2-a]-[1,3,5]triazin-2,4-dion

2-(4-Methoxy-phenyl)-6H,8H-imidazo[1,2-a]pyrimidin-5,7-dion

2-(4-Methylsulfonamido-phenyl)-6H,8H-imidazo[1,2-a]pyrimidin-5,7-dion

6-(4-Methoxy-phenyl)-1H,3H-imidazo[2,1-f][1,2,4]triazin-2,4-dion

6-(4-Methylsulfonyloxy-phenyl)-1H,3H-imidazo[2,1-f][1,2,4]-triazin-2,4-dion

Beispiel A

Tabletten zu 100 mg 7-(4-Methoxy-phenyl)-1H,3H-imidazo-[1,2-a][1.3.5]triazin-2,4-dion

Zusammensetzung
1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Feuchtsiebung: 1,5 mm
Trocknen: Umlufttrockenschrank 50°C
Trockensiebung: 1 mm
Dem Granulat die restlichen Hilfsstoffe zumischen und Endmischung zu Tabletten verpressen.
Tablettengewicht: 175 mg
Stempel: 8 mm

Beispiel B

Dragées zu 50 mg 7-(4-Methoxy-phenyl)-1H,3H-imidazo-[1,2-a][1.3.5]triazin-2,4-dion

Zusammensetzung:
1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wäßriger Lösung der löslichen Stärke gleichmäßig befeuchten.

Feuchtsiebung: 1,0 mm

Trockensiebung: 1,0 mm,

Trocknung: 50°C im Umlufttrockenschrank

Granulat und restliche Hilfsstoffe mischen und zu Kernen verpressen.

Kerngewicht: 80 mg

Stempel: 6 mm

Wölbungsradius: 5 mm

Die fertigen Kerne werden auf übliche Weise mit einem Zuckerüberzug im Dragierkessel versehen.

Dragéegewicht: 120 mg

## Beispiel C

Suppositorien zu 75 mg 7-(4-Methoxy-phenyl)-1H,3H-imidazo-[1,2-a][1.3.5]triazin-2,4-dion

1 Zäpfchen enthält:

| | |
|---|---|
| Wirksubstanz | 75,0 mg |
| Zäpfchenmasse (z.B. Witepsol H 19 und Witepsol W 45) | 1 625,0 mg |
| | 1 700,0 mg |

## Herstellungsverfahren:

Die Zäpfchenmasse wird geschmolzen. Bei 38°C wird die gemahlene Wirksubstanz in der Schmelze homogen dispergiert. Es wird auf 35°C abgekühlt und in vorgekühlte Suppositorienformen ausgegossen.

Zäpfchengewicht: 1,7 g

Beispiel D

Ampullen zu 50 mg 7-(4-Methoxy-phenyl)-1H,3H-imidazo-[1,2-a][1.3.5]triazin-2,4-dion

1 Ampulle enthält:

| | |
|---|---|
| Wirksubstanz | 50,0 mg |
| Ethoxylierte Hydroxystearinsäure | 250,0 mg |
| 1,2-Propylenglykol | 1000,0 mg |
| Dest. Wasser ad | 5,0 ml |

Herstellungsverfahren:

Die Wirksubstanz wird in 1,2-Propylenglykol und ethoxylierter Hydroxystearinsäure gelöst, dann mit Wasser auf das angegebene Volumen aufgefüllt und steril filtriert.

Abfüllung: in Ampullen zu 5 ml

Sterilisation: 20 Minuten bei 120°C

Beispiel E

Tropfen zu 100 mg 7-(4-Methoxy-phenyl)-1H,3H-imidazo-[1,2-a][1.3.5]triazin-2,4-dion

| | | |
|---|---|---|
| Wirksubstanz | | 1,0 g |
| p-Oxybenzoesäuremethylester | | 0,035 g |
| p-Oxybenzoesäurepropylester | | 0,015 g |
| Anisöl | | 0,05 g |
| Menthol | | 0,06 g |
| Saccharin-Natrium | | 1,0 g |
| Glycerin | | 10,0 g |
| Ethanol | | 40,0 g |
| Dest. Wasser | ad | 100,0 ml |

Herstellungsverfahren:

Die Benzoesäureester werden in Ethanol gelöst und anschließend das Anisöl und das Menthol zugegeben. Dann wird die Wirksubstanz, Glycerin und Saccharin-Natrium im Wasser gelöst zugegeben. Die Lösung wird anschließend klar filtriert.

0185346

## Patentansprüche

1. Imidazoderivate der allgemeinen Formel

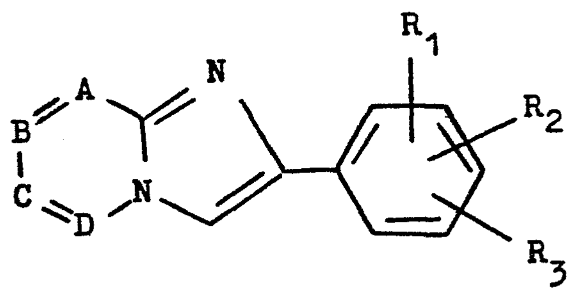

,(I)

in der

einer oder zwei der Reste A, B, C oder D ein Stickstoffatom,

ein weiterer der Reste A, B, C oder D eine Hydroxymethingruppe und

die übrigen der Reste A, B, C oder D Methingruppen, wobei eine dieser Methingruppen, sofern sie neben einem Stickstoffatom steht, durch eine Hydroxymethingruppe oder durch eine durch eine Alkylmercaptogruppe substituierte Methingruppe ersetzt sein kann,

$R_1$ und $R_2$ zusammen mit zwei dazwischen liegenden Kohlenstoffatomen des Phenylringes einen gegebenenfalls durch eine Alkoxygruppe substituierten Phenylring und

$R_3$ ein Wasserstoffatom oder eine Alkoxygruppe oder

einer der Reste $R_1$, $R_2$ oder $R_3$ eine Hydroxy-, Phenylalkoxy-, Alkylmercapto-, Alkylsulfinyl-, Amino-, Alkylsulfonyloxy-, Sulfamyl-, Alkylaminosulfonyl-, Dialkylaminosulfonyl-, Alkylsulfonamido-, N-Alkyl-alkylsulfonamido-, Cyano-, Aminocarbonyl-, Alkylaminocarbonyl- oder Dialkylaminocarbonylgruppe oder,

wenn $R_2$ und $R_3$ nicht gleichzeitig Wasserstoffatome darstellen oder

wenn A, B, C und D zusammen mit dem Imidazolring keine Imidazo[1,2-b]pyridazin-6(5H)-one, Imidazo[1,2-c]pyrimidin-5(6H)-one und 5-Alkylmercapto-imidazo[1,2-c]pyrimidin-7(8H)-one darstellen, auch eine Alkoxy- oder Alkylsulfonylgruppe,

ein zweiter der Reste $R_1$, $R_2$ oder $R_3$ ein Wasserstoffatom, eine Hydroxy- oder Alkoxygruppe und

der letzte der Reste $R_1$, $R_2$ oder $R_3$ ein Wasserstoffatom oder eine Alkoxygruppe, wobei der Alkylteil bei allen vorstehend erwähnten Resten 1 oder 2 Kohlenstoffatome enthalten kann, deren Tautomere und deren Säureadditionssalze.

2. Imidazo[1.2-a]pyrimidin-7-one und -5,7-dione, Imidazo-[1.2-c]pyrimidin-5-one und -7-one, Imidazo[2.1-f][1.2.4]triazin-2-one, -4-one und -2,4-dione, Imidazo[1.2-b]pyridazin-6-one, Imidazo[1.2-a][1.3.5]triazin-2-one, -4-one und -2,4-dione, Imidazo[1.2-a]pyrazin-6-one und -8-one, Imidazo-[1.2-d][1.2.4]triazin-5-one, -8-one und -5,8-dione, Imidazo-[1.2-b][1.2.4]triazin-2-one und -3-one sowie Imidazo[2.1-c]-[1.2.4]triazin-3-one der allgemeinen Formel I gemäß Anspruch 1, deren Tautomere und deren Säureadditionssalze.

3. Imidazoderivate der allgemeinen Formel I gemäß Anspruch 1, in der

einer oder zwei der Reste A, B, C oder D ein Stickstoffatom,

ein weiterer der Reste A, B, C oder D eine Hydroxymethingruppe und

die übrigen der Reste A, B, C oder D Methingruppen, wobei eine dieser Methingruppen, sofern sie neben einem Stickstoffatom steht, durch eine Hydroxymethingruppe ersetzt sein kann,

$R_1$ und $R_2$ zusammen mit zwei dazwischenliegenden Kohlenstoffatomen des Phenylringes einen Phenyl- oder Methoxyphenylring und $R_3$ ein Wasserstoffatom oder eine Methoxygruppe oder

einer der Reste $R_1$, $R_2$ oder $R_3$ eine Hydroxy-, Benzyloxy-, Methylmercapto-, Methylsulfinyl-, Methylsulfonyl-, Amino-, Methylsulfonyloxy-, Methylsulfonamido-, N-Methylmethylsulfonamido-, Sulfamyl-, Methylaminosulfonyl-, Dimethylaminosulfonyl-, Cyano-, Aminocarbonyl-, Methylaminocarbonyl- oder Dimethylaminocarbonylgruppe oder,

wenn $R_2$ und $R_3$ nicht gleichzeitig Wasserstoffatome bedeuten oder

wenn A, B, C und D zusammen mit dem Imidazolring keine Imidazo[1,2-b]pyridazine-6(5H)-one und Imidazo[1,2-c]pyrimidin-5(6H)-one darstellen,
auch eine Methoxygruppe,

ein zweiter der Reste $R_1$, $R_2$ oder $R_3$ ein Wasserstoffatom oder eine Methoxygruppe und

der letzte der Reste $R_1$, $R_2$ oder $R_3$ ein Wasserstoffatom oder eine Methoxygruppe bedeuten, insbesondere jedoch diejenigen Verbindungen, in denen $R_1$ in 4-Position und $R_2$ in 2-Position steht, deren Tautomere und deren Säureadditionssalze.

4. Imidazoderivate der allgemeinen Formel I gemäß Anspruch 1, in der

A, B, C oder D wie im Anspruch 2 definiert sind,

$R_1$ in 4-Stellung eine Cyano-, Dimethylaminocarbonyl-, Methylsulfonyloxy-, Methylsulfonamido-, N-Methyl-methylsulfonamido-, Sulfamyl-, Methylaminosulfonyl- oder Dimethylaminosulfonylgruppe, oder,

wenn $R_2$ und $R_3$ nicht gleichzeitig Wasserstoffatome bedeuten oder

wenn A, B, C und D zusammen mit dem Imidazolring keine Imidazo[1,2-b]pyridazine-6(5H)-one und Imidazo[1,2-c]pyrimidin-5(6H)-one darstellen,

auch eine Methoxygruppe,

$R_2$ in 2-Stellung eine Methoxygruppe und

$R_3$ ein Wasserstoffatom darstellen, deren Tautomere und deren Säureadditionssalze.

5. 2-(2-Methoxy-4-methylsulfonyloxy-phenyl)-8H-imidazo-[1,2-a]pyrimidin-7-on, dessen Tautomere und dessen Säureadditionssalze.

6. 2-(2-Methoxy-4-cyano-phenyl)-8H-imidazo[1,2-a]pyrimidin-7-on, dessen Tautomere und dessen Säureadditionssalze.

7. 2-(2-Methoxy-4-methylsulfonyloxy-phenyl)-7H-imidazo-[1,2-a]pyrazin-8-on, dessen Tautomere und dessen Säureadditionssalze.

8. Physiologisch verträgliche Säureadditionssalze mit anorganischen oder organischen Säuren der Verbindungen gemäß den Ansprüchen 1 bis 7.

9. Arzneimittel, enthaltend eine Verbindung gemäß den Ansprüchen 1 bis 7 oder ein physiologisch verträgliches Säureadditionssalz gemäß Anspruch 8 neben einem oder mehreren inerten Trägerstoffen und/oder Verdünnungsmitteln.

10. Verwendung einer Verbindung gemäß den Ansprüchen 1 bis 7 oder eines physiologisch verträglichen Säureadditionssalzes gemäß Anspruch 8 zur Behandlung von Herzinsuffizienzen.

11. Verfahren zur Herstellung eines Arzneimittels, dadurch gekennzeichnet, daß auf nichtchemischem Wege eine Verbindung gemäß den Ansprüchen 1 bis 7 oder ein physiologisch verträgliches Säureadditionssalz gemäß Anspruch 8 in einen oder mehrere inerte Trägerstoffe und/oder Verdünnungmittel eingearbeitet wird.

12. Verfahren zur Herstellung der neuen Imidazoderivate der allgemeinen Formel I gemäß den Ansprüchen 1 bis 8, dadurch gekennzeichnet, daß

a) eine Verbindung der allgemeinen Formel

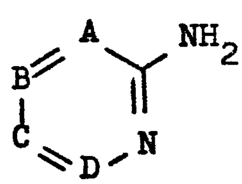

,(II)

in der
A, B, C und D wie in den Ansprüchen 1 bis 7 definiert sind, mit einer Verbindung der allgemeinen Formel

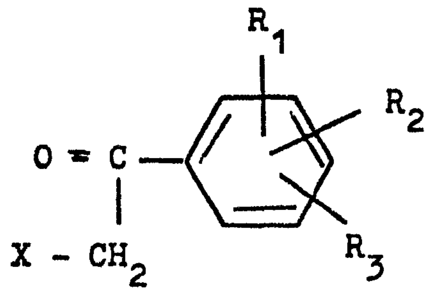

,(III)

in der

$R_1$, $R_2$ und $R_3$ wie in den Ansprüchen 1 bis 7 definiert sind und

X eine nukleophile Austrittsgruppe wie ein Halogenatom darstellt, umgesetzt wird oder

b) zur Herstellung von Verbindungen der allgemeinen Formel I, in der einer der Reste $R_1$, $R_2$ oder $R_3$ eine Alkylsulfonyloxy-, Alkylsulfonamido- oder N-Alkyl-alkylsulfonamidogruppe darstellt, eine Verbindung der allgemeinen Formel

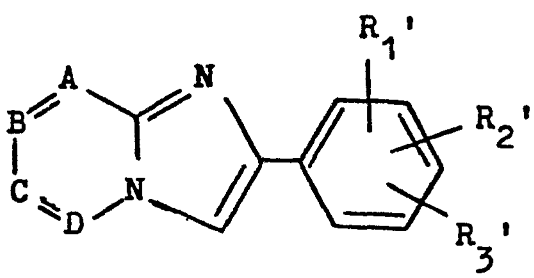

,(IV)

in der

A, B, C und D wie in den Ansprüchen 1 bis 7 definiert sind und

die Reste $R_1'$, $R_2'$ und $R_3'$ mit Ausnahme der Alkylsulfonyloxy-, Alkylsulfonamido- und N-Alkyl-alkylsulfonamidogruppe die für $R_1$, $R_2$ oder $R_3$ in den Ansprüchen 1 bis 7 erwähnten Bedeutungen besitzen, wobei jedoch einer der Reste $R_1'$, $R_2'$ oder $R_3'$ eine Hydroxy-, Amino-, Methylamino- oder Ethylaminogruppe darstellen muß, mit einer Verbindung der allgemeinen Formel

$R_4 - SO_2 - Y$ ,(V)

in der
$R_4$ eine Methyl- oder Ethylgruppe und
Y eine nukleophile Austrittsgruppe wie ein Halogenatom oder eine Alkoxygruppe bedeuten, umgesetzt wird oder

c) zur Herstellung von Verbindungen der allgemeinen Formel I, in der mindestens einer der Reste $R_1$, $R_2$ oder $R_3$ eine Alkoxy-, Phenylalkoxy- oder Alkylmercaptogruppe oder einer der Reste $R_1$, $R_2$ oder $R_3$ eine N-Alkyl-alkylsulfonamidogruppe darstellen, eine Verbindung der allgemeinen Formel

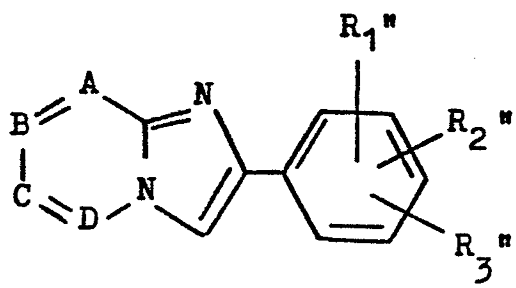

,(VI)

in der
A, B, C und D wie in den Ansprüchen 1 bis 7 definiert sind und
$R_1$", $R_2$" und $R_3$" die für $R_1$, $R_2$ oder $R_3$ in den Ansprüchen 1 bis 7 erwähnten Bedeutungen besitzen, wobei jedoch mindestens einer der Reste $R_1$", $R_2$" oder $R_3$" eine Hydroxy-, Mercapto- oder Alkylsulfonamidogruppe darstellen muß, mit einer Verbindung der allgemeinen Formel

$R_4 - Z$ ,(VII)

in der
$R_4$ eine Alkyl- oder Phenylalkylgruppe mit jeweils 1 oder 2 Kohlenstoffatomen im Alkylteil und

Z eine nukleophile Austrittsgruppe wie ein Halogenatom oder eine Sulfonyloxygruppe darstellen, alkyliert wird oder

d) zur Herstellung von Verbindungen der allgemeinen Formel I, in der einer der Reste $R_1$, $R_2$ oder $R_3$ eine Aminocarbonyl-, Alkylaminocarbonyl-, Dialkylaminocarbonyl-, Sulfamyl-, Alkylaminosulfonyl- oder Dialkylaminosulfonylgruppe darstellt, eine Verbindung der allgemeinen Formel

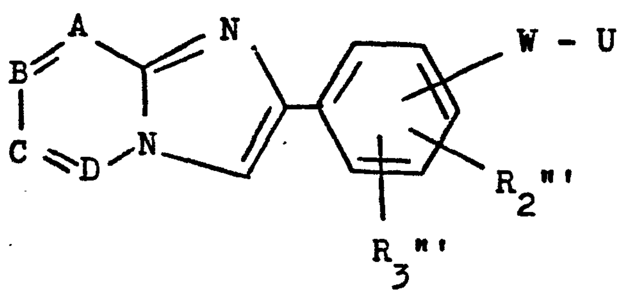

,(VIII)

in der
A, B, C und D wie in den Ansprüchen 1 bis 7 definiert sind,
$R_2'''$ und $R_3'''$ die für $R_1$, $R_2$ und $R_3$ mit Ausnahme der Aminocarbonyl-, Alkylaminocarbonyl-, Dialkylaminocarbonyl-, Sulfamyl-, Alkylaminosulfonyl- oder Dialkylaminosulfonylgruppe in den Ansprüchen 1 bis 7 erwähnten Bedeutungen besitzen,
W eine Carbonyl- oder Sulfonylgruppe und
U eine nukleophile Austrittsgruppe wie ein Halogenatom oder eine Alkoxygruppe darstellen, mit einem Amin der allgemeinen Formel

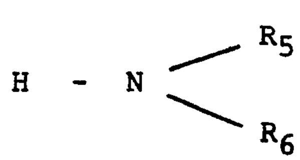

,(IX)

in der
$R_5$ und $R_6$, die gleich oder verschieden sein können, jeweils ein Wasserstoffatom, eine Methyl- oder Ethylgruppe darstellen, umgesetzt wird oder

e) zur Herstellung von Verbindungen der allgemeinen Formel I, in der die Reste A, B, C und D wie eingangs definiert sind und einer der Reste $R_1$, $R_2$ oder $R_3$ eine Amino-, Hydroxy- oder Aminocarbonylgruppe darstellt oder $R_1$, $R_2$ und $R_3$ wie eingangs definiert sind und mindestens einer der Reste A, B, C oder D eine Hydroxymethingruppe darstellt, eine Verbindung der allgemeinen Formel

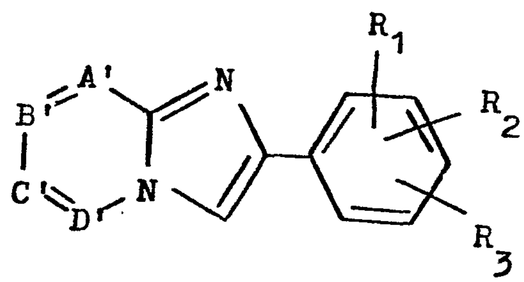
,(X)

in der
$R_1$, $R_2$ und $R_3$ wie in den Ansprüchen 1 bis 7 definiert sind und
A', B', C' und D' die für A, B, C und D in den Ansprüchen 1 bis 7 erwähnten Bedeutungen aufweisen, wobei jedoch entweder eine der in den Ansprüchen 1 bis 7 erwähnten Methingruppen durch einen hydrolytisch abspaltbaren Rest wie durch ein Halogenatom, eine Alkoxy- oder Alkylmercaptogruppe substituiert ist oder einer der Reste $R_1$, $R_2$ oder $R_3$ eine Alkanoylamino-, Cyano- oder Alkylsulfonyloxygruppe darstellen muß, hydrolysiert wird oder

f) zur Herstellung von Verbindungen der allgemeinen Formel I, in der ein oder zwei der Reste A, B, C oder D ein Stickstoffatom, ein weiterer der Reste A, B, C oder D eine Hydroxymethin- oder Alkylmercaptomethingruppe und die übrigen der Reste A, B, C oder D Methingruppen darstellen, ein oder zwei Reste von einer Verbindung der allgemeinen Formel

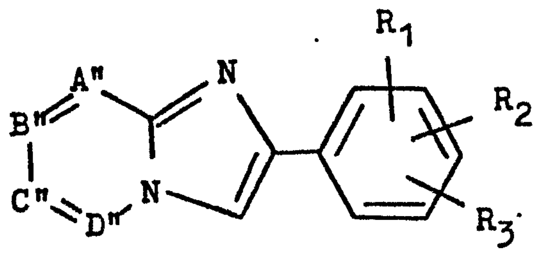

,(XI)

in der
$R_1$, $R_2$ und $R_3$ wie in den Ansprüchen 1 bis 7 definiert sind und
die Reste A", B", C" und D" die für A, B, C und D in den Ansprüchen 1 bis 7 erwähnten Bedeutungen aufweisen, wobei jedoch mindestens eine der in den Ansprüchen 1 bis 7 erwähnten Methingruppen durch einen reduktiv abspaltbaren Rest wie durch ein Halogenatom oder eine Alkylmercaptogruppe substituiert sein muß, reduktiv abgespalten wird und

gewünschtenfalls anschließend eine so erhaltene Verbindung der allgemeinen Formel I, in der einer der Rest $R_1$, $R_2$ oder $R_3$ eine Aminogruppe darstellt, nach Überführung in das entsprechende Diazoniumsalz in eine Verbindung der allgemeinen Formel I, in der einer der Reste $R_1$, $R_2$ oder $R_3$ eine Cyanogruppe darstellt, übergeführt wird oder

eine so erhaltene Verbindung der allgemeinen Formel I in ihr Säureadditionssalz, insbesondere in ihr Säureadditionssalz mit einer physiologisch verträglichen anorganischen oder organischen Säure übergeführt wird.